# EUROPEAN PATENT SPECIFICATION

(11) **EP 0 808 832 B1**
(45) Date of publication and mention of the grant of the patent: **23.04.2003**
(21) Application number: 97107868.8
(22) Date of filing: 14.05.1997
(51) Int. Cl.: C07C 401/00, A61K 31/59

(54) **Fluorinated vitamin D3 analogs**
Fluorinierte Vitamin D3 Analoge
Dérivés fluorinés de Vitamine D3

(30) Priority: 23.05.1996 US 18219; 19.03.1997 US 39901
(43) Date of publication of application: 26.11.1997
(73) Proprietor: F. HOFFMANN-LA ROCHE AG, 4070 Basel (CH)
(72) Inventor: Manchand, Percy Sarwood, Montclair, New Jersey 07042 (US); Nestor Jr., John Joseph, Louisville, Kentucky 40207 (US); Uskokovic, Milan Radoje, Upper Montclair, New Jersey 07043 (US); Vickery, Brian Henry, Mountain View, California 94040 (US)
(74) Representative: Schager, Frank, Dr.

(56) References cited:
- WO-A-93/12081
- WO-A-94/00428
- WO-A-97/16193
- JIAN-YUAN ZHOU ET AL: "DEVELOPMENT OF A NOVEL 1,25(OH)2-VITAMIN D3 ANALOG WITH POTENT ABILITY TO INDUCE HL-60 CELL DIFFERENTIATION WITHOUT MODULATING CALCIUUM METABOLISM" BLOOD, vol. 78, no. 1, 1 July 1991, pages 75-82, XP002025469
- FARACH-CARSON M C ET AL: "Nongenomic actions of 1,25-dihydroxyvitamin D3 in rat osteosarcoma cells: structure-function studies using ligand analogs" ENDOCRINOLOGY, vol. 129, no. 4, 1991, pages 1876-1884, XP002085971 (BALTIMORE)
- NORMAN A W ET AL: "Structure-function studies on analogs of 1.alpha.,25-dihydroxyvitamin D3: differential effects on leukemic cell growth, differentiation, and intestinal calcium absorption" CANCER RESEARCH., vol. 50, no. 21, 1990, pages 6857-6864, XP002085972 MD US
- COLLINS E D ET AL: "1,25-Dihydroxyvitamin D3 analogs: comparison of 1,25-dihydroxyvitamin D3 receptor ligand specificity and biological activity in the chick and human promyelocytic (HL-60) cells" PROCEEDINGS OF THE WORKSHOP ON VITAMIN D, vol. 7, 1988, pages 433-434, XP002085973
- NEEF G ET AL: "20-Methyl vitamin D analogs" PROCEEDINGS OF THE WORKSHOP ON VITAMIN D, vol. 9, 1994, pages 97-98, XP002085974

## Description

This invention relates to fluorinated Vitamin D₃ analogs, both as such and for use as therapeutically active agents, particularly for treating osteoporosis.

Osteoporosis is the most common form of metabolic bone disease and may be considered the symptomatic, fracture stage of bone loss (osteopenia). Although osteoporosis may occur secondary to a number of underlying diseases, 90% of all cases appear to be idiopathic. Postmenopausal women are at risk for idiopathic osteoporosis (postmenopausal or Type I osteoporosis); another particularly high risk group for idiopathic osteoporosis is the elderly of either sex (senile or Type II osteoporosis). Osteoporosis has also been related to corticosteroid use, immobilization or extended bed rest, alcoholism, diabetes, gonadotoxic chemotherapy, hyperprolactinemia, anorexia nervosa, primary and secondary amenorrhea, transplant immunosuppression, and oophorectomy. Postmenopausal osteoporosis is characterized by fractures of the spine, while femoral neck fractures are the dominant features of senile osteoporosis.

Various approaches have been suggested for increasing bone mass in humans afflicted with osteoporosis, including administration of androgens, fluoride salts, and parathyroid hormone and modified versions of parathyroid hormone. It has also been suggested that bisphosphonates, calcitonin, calcium, 1,25-dihydroxy vitamin D₃ and some of its analogs, and/or estrogens, alone or in combination, may be useful for preserving existing bone mass.

Hefti et al., *Clinical Science*, 62:389 (1982), describe studies using a high calcium diet supplemented with either parathyroid hormone or 1,25-(OH)₂ vitamin D₃ using normal and osteoporotic adult rats. The authors report that, although these studies showed an increase of whole-body calcium and skeletal mass, there was no restoration of individual trabeculae lost during the development of osteoporosis. Endo et al., *Nature*, 286:262 (1980), discuss the use of metabolites of vitamin D in conjunction with parathyroid hormone (PTH) to stimulate bone formation in vitro. However, these treatments with PTH and 1,25-(OH)₂ vitamin D₃ were no more effective than PTH alone in stimulating re-calcification of bone.

Rader et al., *Calcified Tissue International*, 29(1):21 (1979), describe the treatment of thyroparathyroidectomized rats with dietary calcium and intraperitoneal injection of a parathyroid extract. Although this treatment stimulated 1,25-(OH)₂ vitamin D₃ production and effected a marked increase in bone mineralization, it was also found to produce bone resorption as evidenced by the appearance of cavities in the cortical bone. There was no effect on rates of bone formation, or bone matrix apposition. Wong et al., *Surgical Forum,* 30:100 (1979), teach the administration to thyroparathyroidectomized dogs of daily intramuscular parathyroid extract or oral 1,25-(OH)₂ vitamin D₃ simultaneously with thyroid replacement therapy. The effect of these treatments on absorption of dietary calcium is discussed in the context of parathyroidism although not in the context of osteoporosis.

Peacock et al., *Vitamin D Proceedings Workshop.,* E. Norman, Ed., p. 411 (1977), disclose the inhibition by calcitonin and steroid sex hormones of the resorptive effect of vitamin D metabolites and parathyroid hormone on mouse calvaria bone in tissue culture. Pechet et al., *American Journal of Medicine,* 43(5):696 (1967), teach that minimum levels of parathyroid hormone are necessary in order for vitamin D to exert its effects on bone resorption rather than bone formation. In Mahgoub et al., *Biochemical and Biophysical Research Communications*, 62:901 (1975), the authors state that active vitamin D metabolites (25-OH vitamin D₃ and 1,25-(OH)₂ vitamin D₃) potentiate the ability of parathyroid hormone to elevate the cyclic AMP levels of cultured rat fetal bone cells.

Vitamin D₃ is a critical element in the metabolism of calcium, promoting intestinal absorption of calcium and phosphorus, maintaining adequate serum levels of calcium and phosphorus, and stimulating flux of calcium into and out of bone. The D vitamins are hydroxylated *in vivo*, with the resulting 1a,25-dihydroxy metabolite being the active material. Animal studies with 1,25-(OH)₂ vitamin D have suggested bone anabolic activity. Aerssens et al. in *Calcif Tissue Int,* **55**:443-450 (1994) reported upon the effect of 1a-hydroxy Vitamin D₃ on bone strength and composition in growing rats with and without corticosteroid treatment. However, human usage is restricted to antiresorption due to the poor therapeutic ratio (hypercalciuria and hypercalcemia as well as nephrotoxicity).

Dechant and Goa, in "Calcitriol. A review of its use in the treatment of postmenopausal osteoporosis and its potential in corticosteroid-induced osteoporosis", *Drugs Aging* (NEW ZEALAND) **5**(4):300-17 (1994), reported that 1,25-dihydroxyvitamin D₃ (calcitriol) has shown efficacy in the treatment of postmenopausal osteoporosis (and promise in corticosteroid-induced osteoporosis) based upon a clinical trial in 622 women with postmenopausal osteoporosis. Patients with mild to moderate disease (but not those with more severe disease) who received calcitriol (0.25 microgram twice daily) had a significant 3-fold lower rate of new vertebral fractures after 3 years of treatment, compared with patients receiving elemental calcium 1000 mg/day. In patients commencing long term treatment with prednisone or prednisolone, calcitriol 0.5 to 1.0 micrograms/day plus calcium 1000 mg/day, administered with or without intranasal calcitonin 400 IU/day, prevented steroid-induced bone loss. Overall, calcitriol was well tolerated. At recommended dosages hypercalcaemia was infrequent and mild, generally responding to reductions in calcium intake and/or calcitriol dosage. The narrow therapeutic window of calcitriol required that its use be adequately supervised, with periodic monitoring of serum calcium and creatinine levels. This study clearly identifies the key limitation of calcitriol therapy as the close proximity of therapeutic and toxic doses.

Baggiolini et al. in European Patent Publication No. 580,968 disclose fluorinated vitamin D₃ analogs, including 1a- fluoro-25-hydroxy-16-ene-23-yne-26,27-hexalluorochole- calciferol, useful for the treatment of hyperproliferative disorders of the skin, for the treatment of cancer and leukemia, and for the treatment of sebaceous gland diseases. US-A-5,696,103 (published on December 1997) discloses and claims the use of this compound for the restoration of bone mass and/or density in osteoporosis.

The 1a-fluoro analogs of Vitamin D₃ disclosed herein have not previously been described, nor has their use in the treatment of osteoporosis been recognized.

This invention provides fluorinated Vitamin D₃ analogs of the Formula (I): 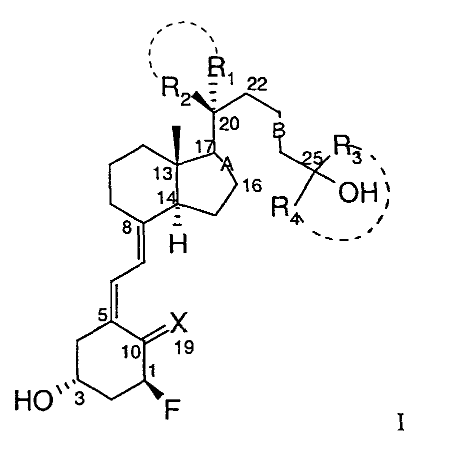 wherein:
X is (H,H) or =CH₂;
R₁ and R₂ together with C-20 form a cyclopropano, cyclodifluoropano or cyclotetrafluoropropano group
R₃ and R₄ are, independently of each other, selected from the group consisting of CH₃, CF₃, CH₂ CH₃, CH₂ CF₃, CF₂ CH₃ and CF₂ CF₃;
A is a single bond or a double bond; and
B is a double bond or a triple bond;

This invention also provides compositions comprising a pharmaceutically acceptable carrier and a fluorinated Vitamin D₃ analog of Formula **(I)**, as defined above, as well as the use of these compounds in the manufacture of medicaments for treating osteoporosis.

The present invention further relates to the use of a compound of Formula **(I),** wherei X is =CH₂, one of R₁ and R² is hydrogen, and the other is (C₁-C₄) alkyl R₃ and R₄ are (C₁-C₄) alkyl, A is a double bond and B is a double bond, for the manufacture of a medicament for treating osteoporosis. Such a medicament can be administered in an amount therapeutically effective to restore bone density to an asymptomatic level, without inducing hypercalciuria, hypercalcemia, or nephrotoxicity.

The preparations of compounds of Formula **(I)** and the key intermediates required for their synthesis are illustrated by the following figures:
*Figures 1, 2 & 2a* illustrate the synthesis of the 20-methyl analog (Ia) of 1a-fluoro-25-hydroxy-23-yne-26,27-hexafluoro-cholecalciferol and intermediates (IIa) and (VIIIa) used in the preparation of (Ia), respectively.
*Figures 3, 4 & 4a* illustrate the synthesis of the 20-cyclopropyl analog (Ib) of 1a-fluoro-25-hydroxy-23-yne-26,27-hexafluoro-cholecalciferol and intermediates (IIb) and (Xb) used in the preparation of (Ib), respectively.
*Figures 5, 6 & 6a* illustrate the synthesis of the 20-ene analog (Ic) of 1a-fluoro-25-hydroxy-23-yne-26,27-hexafluorocholecalciferol and intermediates (IIc) and (XIc) used in the preparation of (Ic), respectively.
*Figures 7 & 8* illustrate the synthesis of the 20-methyl-26,27-bishomo-26,26a,27,27a-decafluoro analog (Id) of 1a-fluoro-25-hydroxy-23-yne-cholecalciferol and intermediates (IId) used in the preparation of (Id), respectively.
*Figures 9 & 10* illustrate the synthesis of the 20-methyl analog (Ie) of 1a-fluoro-25-hydroxy-16-ene-23-yne-26,27-hexafluoro-cholecalciferol and intermediates (IIe) used in the preparation of (Ie), respectively.
*Figures 11 & 12* illustrate the synthesis of the 20-cyclopropyl analog (If) of 1a-fluoro-25-hydroxy-16-ene-23-yne-26,27-hexafluoro-cholecalciferol and intermediates (IIf) used in the preparation of (If), respectively.
*Figures 13 & 14* illustrate the synthesis of the 20-methyl-26,27-bishomo-26a,27a-hexafluoro analog (Ig) of 1a-fluoro-25-hydroxy-16-ene-23-yne-cholecalciferol and intermediates (IIg) used in the preparation of (Ig), respectively.
*Figure 15* illustrates the synthesis of key intermediates (IIh), (IIi), (IIj), (IIk), (IIl), (IIm) and (IIn).
*Figure 16* illustrates the synthesis of the 20-methyl analog (Ih) of 1a-fluoro-25-hydroxy-23Z-ene-26,27-hexafluorocholecalciferol.
*Figure 17* illustrates the synthesis of the 20-cyclopropyl analog (Ii) of 1a-fluoro-25-hydroxy-23Z-ene-26,27-hexafluorocholecalciferol.
*Figure 18* illustrates the synthesis of the 20-ene-analog (Ij) of 1a-fluoro-25-hydroxy-23Z-ene-26,27-hexafluorocholecalciferol.
*Figure 19* illustrates the synthesis of the 20-methyl-26,27-bishomo-26,26a,27,27a-decafluoro analog (Ik) of 1a-fluoro-25-hydroxy-23Z-ene-cholecalciferol.
*Figure 20* illustrates the synthesis of the 20-methyl analog (Il) of 1a-fluoro-25-hydroxy-16,23Z-diene-26,27-hexafluoro-cholecalciferol.
*Figure 21* illustrates the synthesis of the 20-cyclopropyl analog (Im) of 1a-fluoro-25-hydroxy-16,23Z-diene-26,27-hexafluoro-cholecalciferol.
*Figure 22* illustrates the synthesis of the 20-methyl-26,27-bishomo-26a,27a-hexafluoro analog (In) of 1a-fluoro-25-hydroxy-16,23Z-diene-cholecalciferol.
*Figure 23* illustrates the synthesis of key intermediates (IIo), (IIp), (IIq), (IIr), (IIs), (IIt) and (IIu).
*Figure 24* illustrates the synthesis of the 20-methyl analog (Io) of 1a-fluoro-25-hydroxy-23E-ene-26,27-hexafluorocholecalciferol.
*Figure 25* illustrates the synthesis of the 20-cyclopropyl analog (Ip) of 1a-fluoro-25-hydroxy-23E-ene-26,27-hexafluorocholecalciferol.
*Figure 26* illustrates the synthesis of the 20-ene analog (Iq) of 1a-fluoro-25-hydroxy-23E-ene-26,27-hexafluorocholecalciferol.
*Figure 27* illustrates the synthesis of the 20-methyl 26,27-bishomo-26,26a,27,27a-decafluoro analog (Ir) of 1a-fluoro-25-hydroxy-23E-ene-cholecalciferol.
*Figure 28* illustrates the synthesis of the 20-methyl analog (Is) of 1a-fluoro-25-hydroxy-16,23E-diene-26,27-hexafluoro-cholecalciferol.
*Figure 29* illustrates the synthesis of the 20-cyclopropyl analog (It) of 1a-fluoro-25-hydroxy-16,23E-diene-26,27-hexafluoro-cholecalciferol.
*Figure 30* illustrates the synthesis of the 20-methyl-26,27-bishomo-26a,27a-hexafluoro analog (Iu) of 1a-fluoro-25-hydroxy-16,23E-diene-cholecalciferol.
*Figures 31, 32 & 32a* illustrate the synthesis of the 20-ene analog (Iv) of 1a-fluoro-25-hydroxy-16-ene-23-yne-26,27-hexafluoro-cholecalciferol and intermediates (IIv) and (XXXIXv) used in the preparation of (Iv), respectively.
*Figures 33 & 34* illustrate the synthesis of the 20-ene analog (Iw) of 1a-fluoro-25-hydroxy-16,23Z-diene-26,27-hexafluoro-cholecalciferol and intermediates (IIw) used in the preparation of (Iw), respectively.
*Figures 35 & 36* illustrate the synthesis of the 20-ene analog (Iz) of 1a-fluoro-25-hydroxy-16,23Z-diene-26,27-hexafluoro-cholecalciferol and intermediates (IIz) used in the preparation of (Iz), respectively.
*Figure 37* illustrates the synthesis of the precursor (3R-(3a,5β,Z)]-2 [2-[3-fluoro-5-[[(1,1-dimethylethyl)-dimethylsilyl]oxy]cyclohexylidene]ethyl] diphenyl phosphine oxide (**15**) used in the synthesis of 1a-fluoro-19-nor Vitamin D.

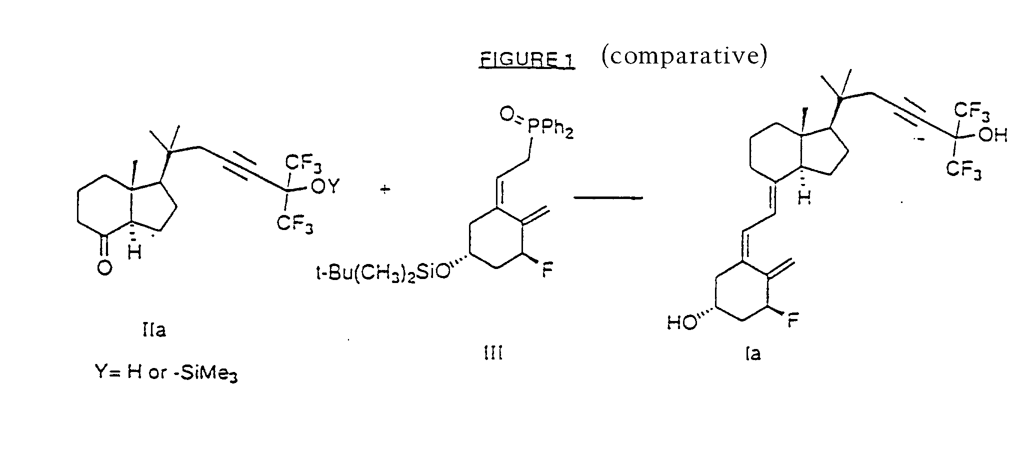

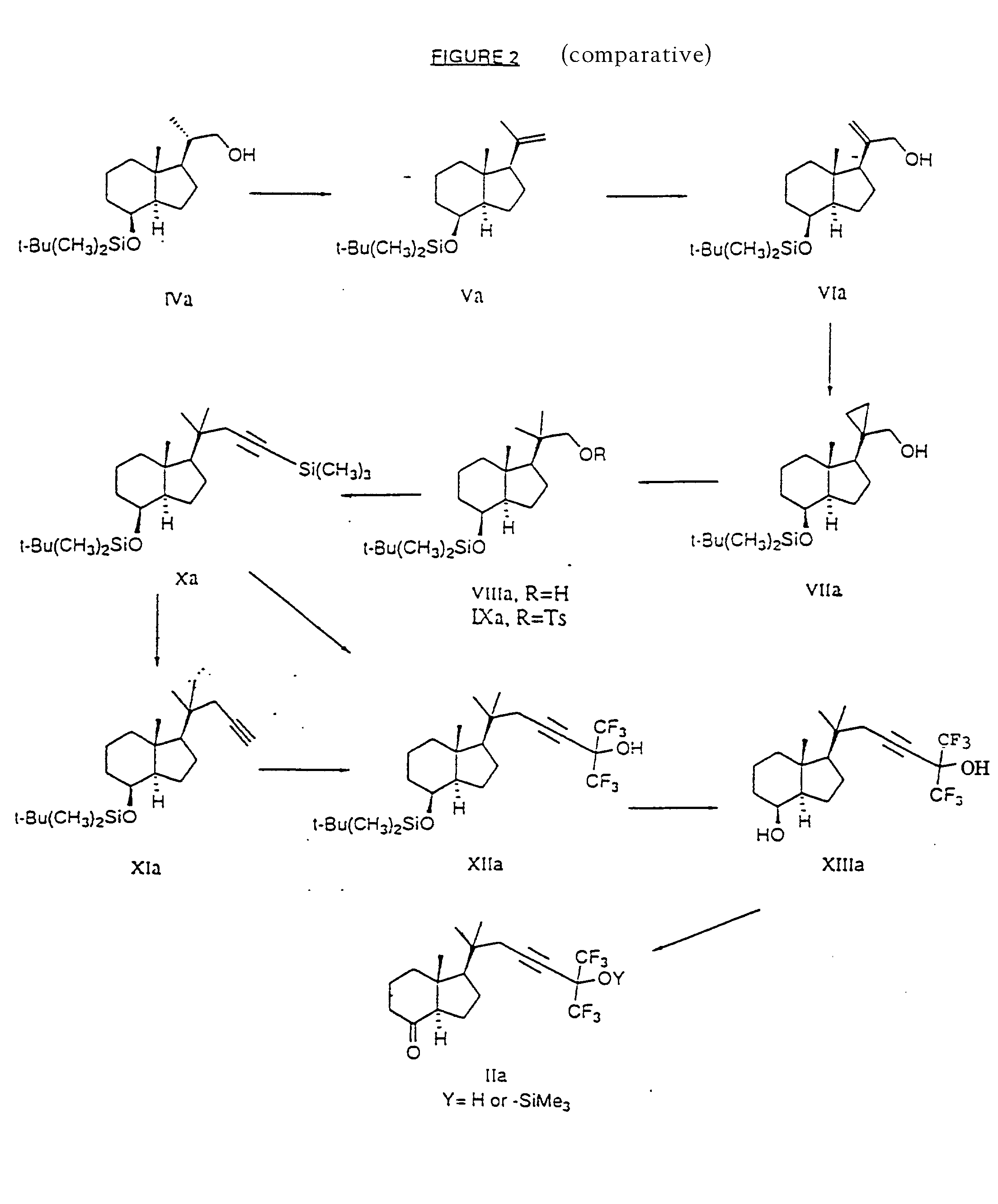

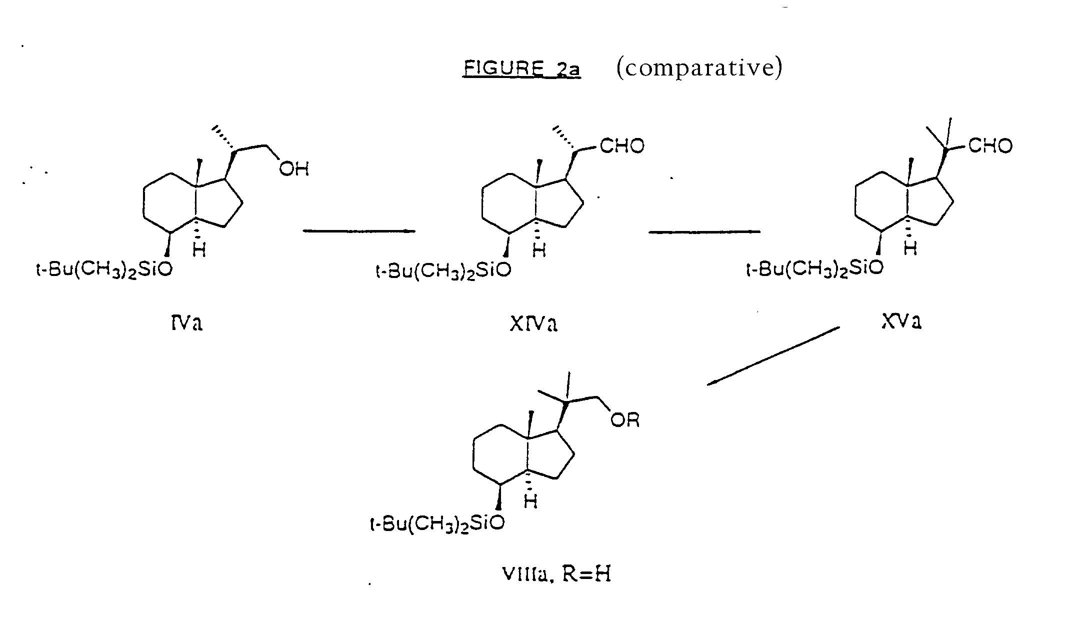

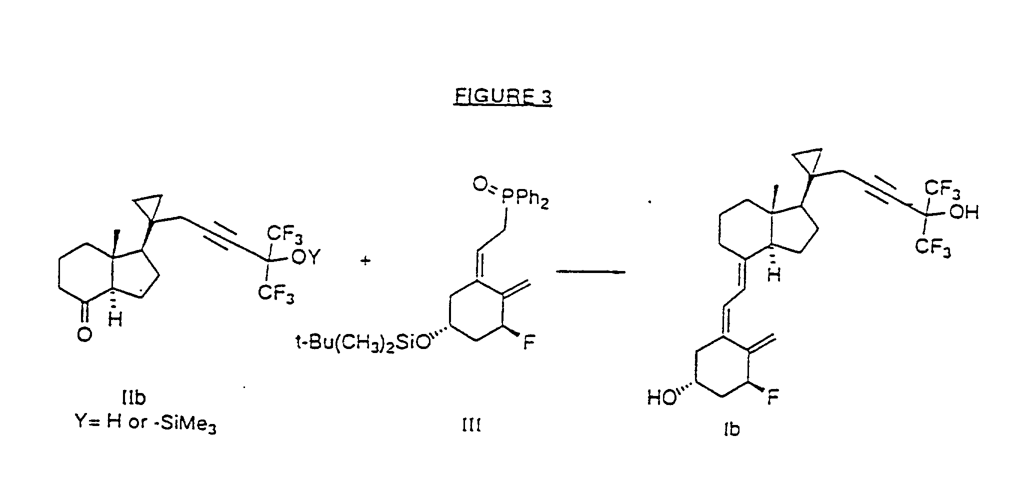

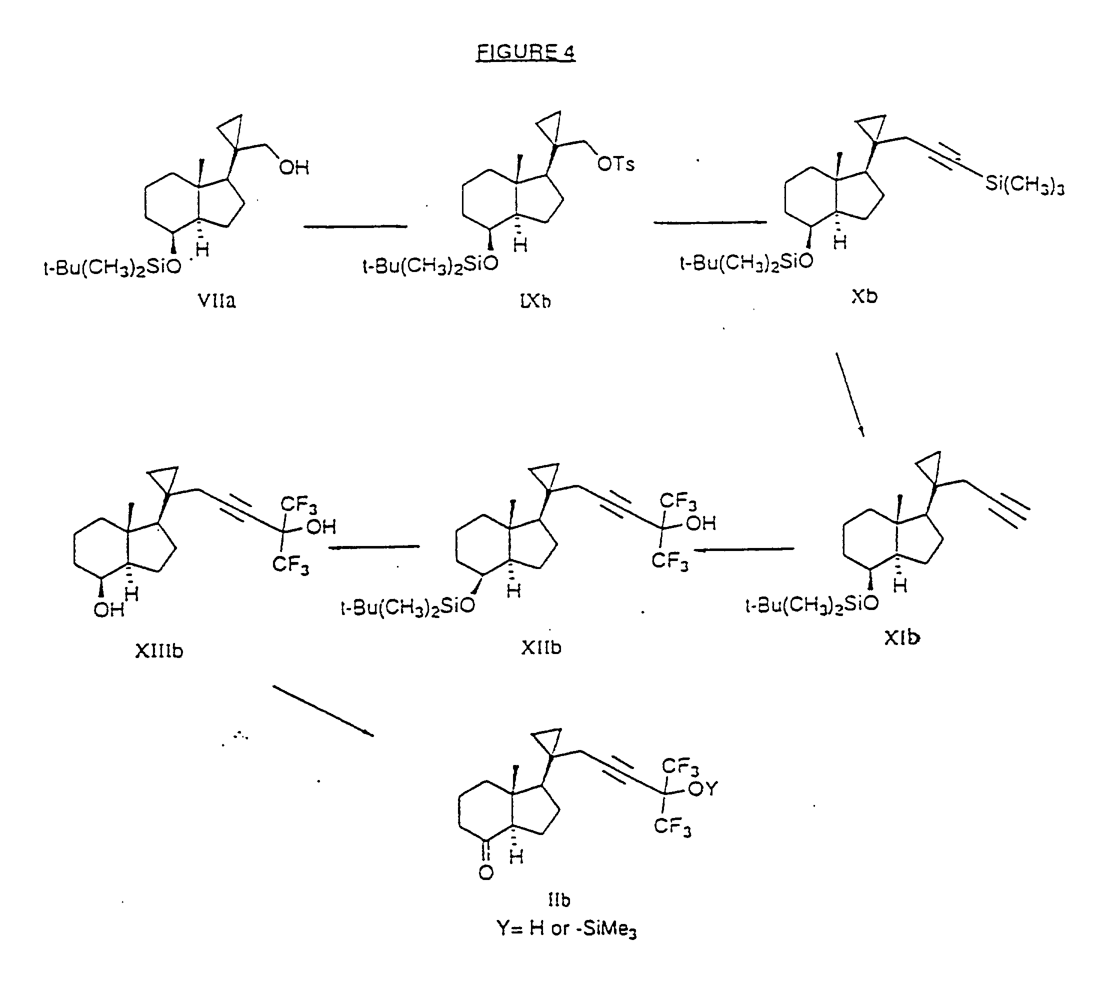

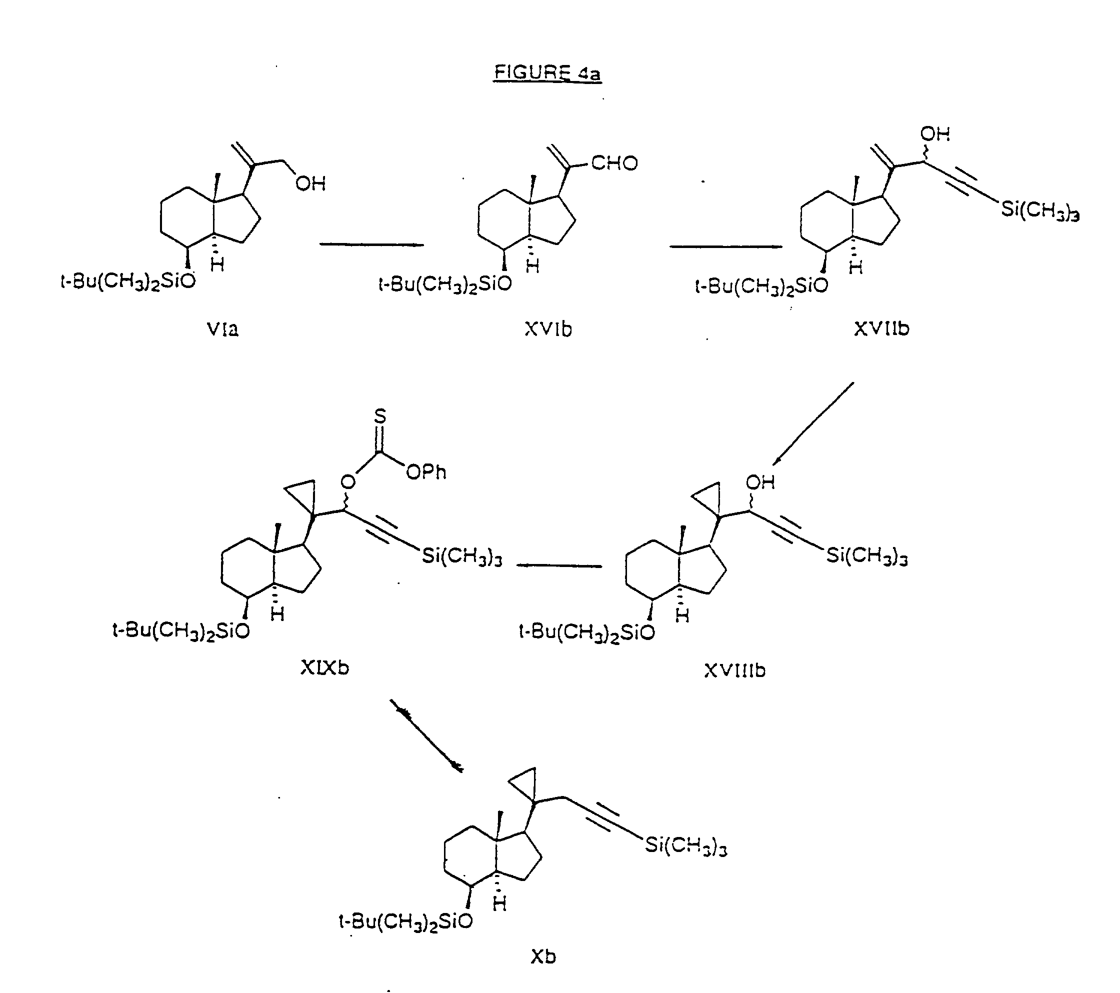

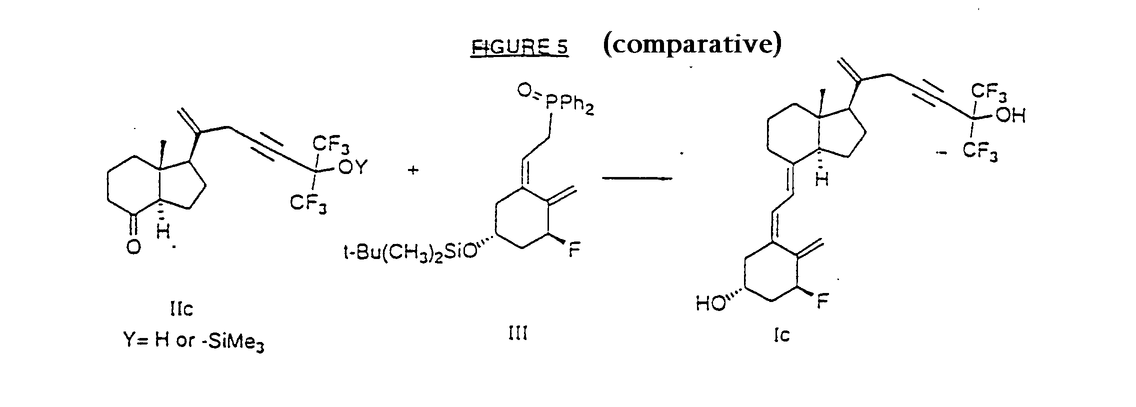

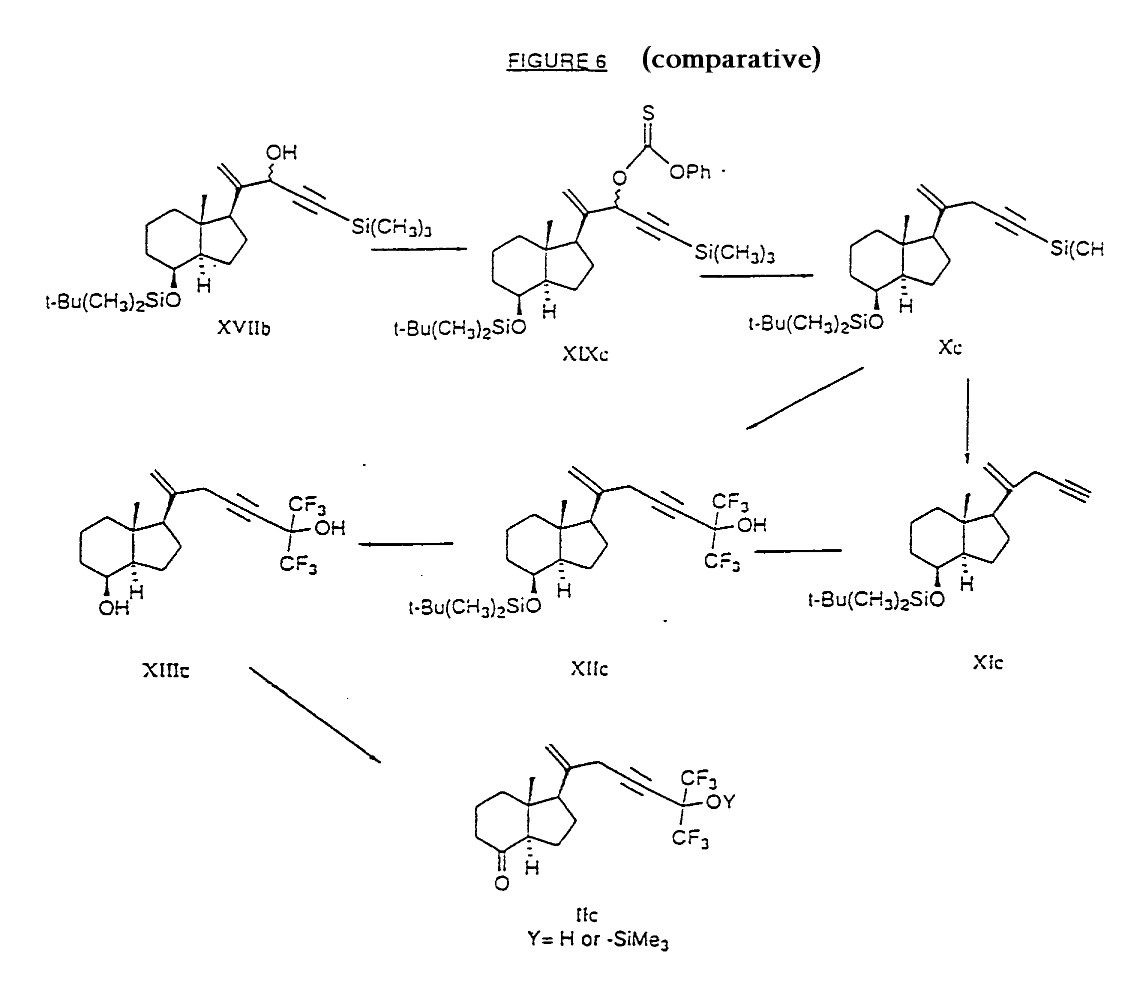

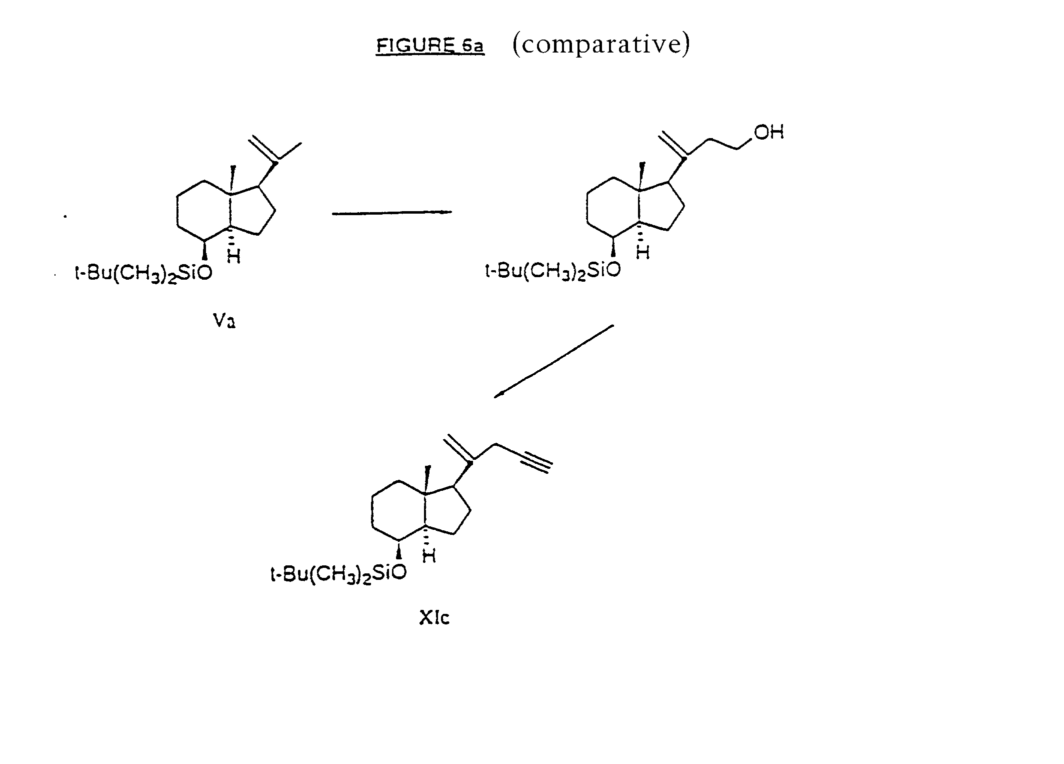

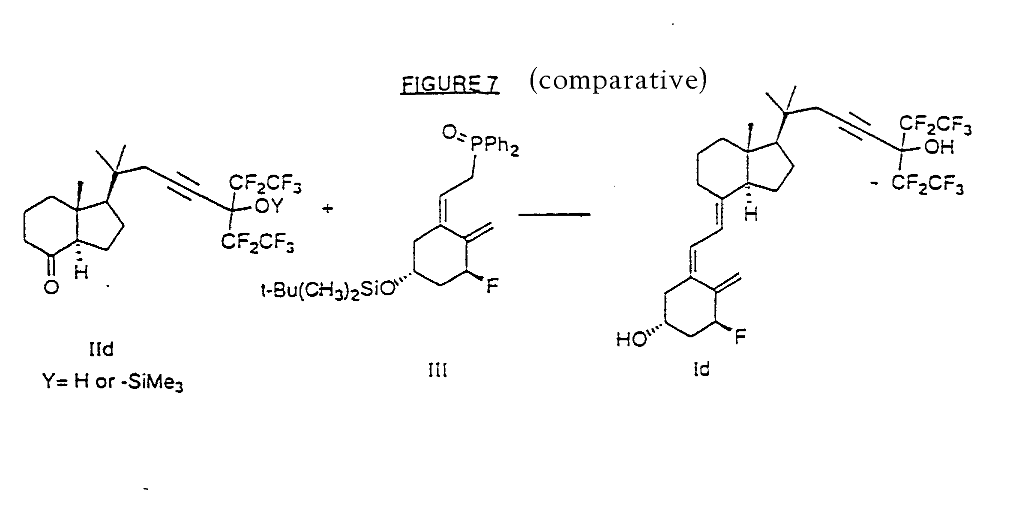

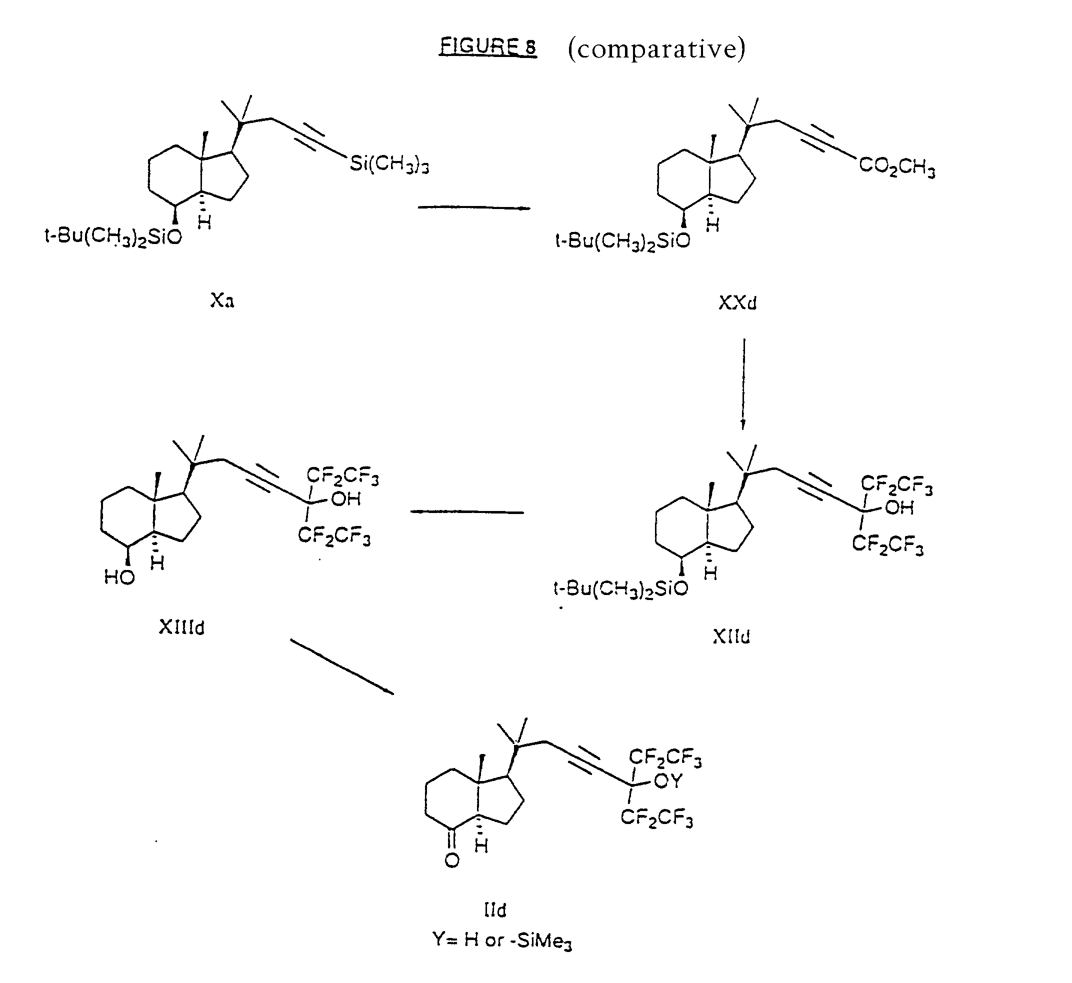

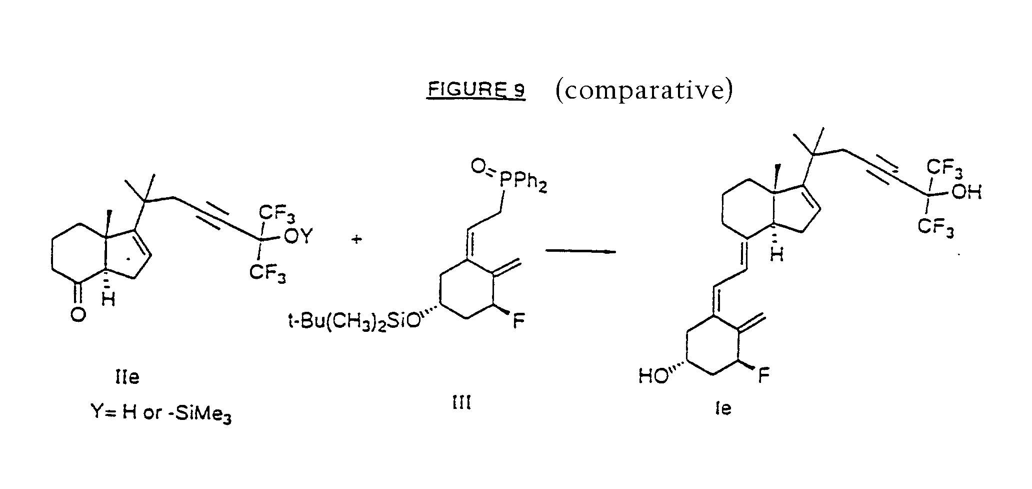

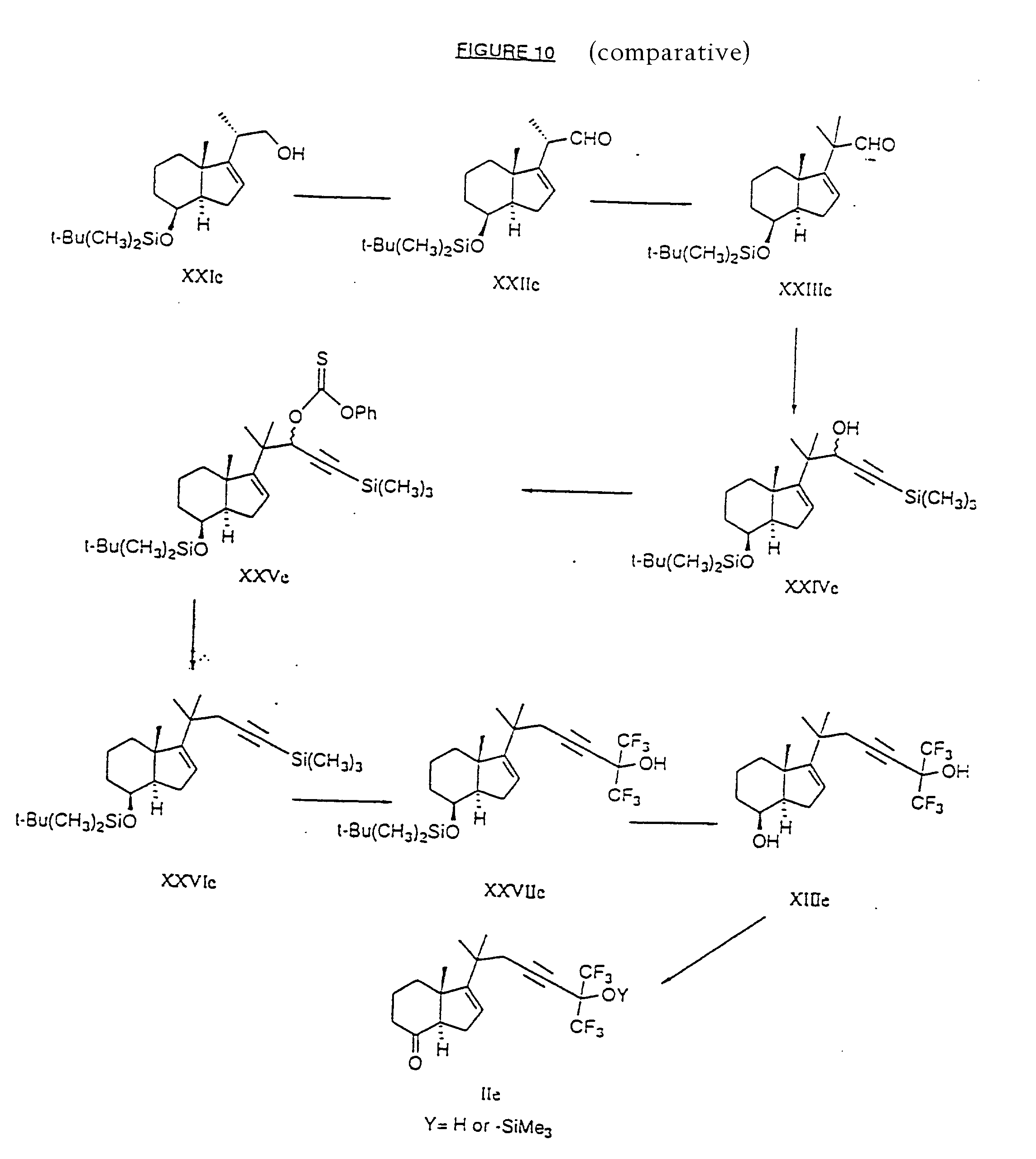

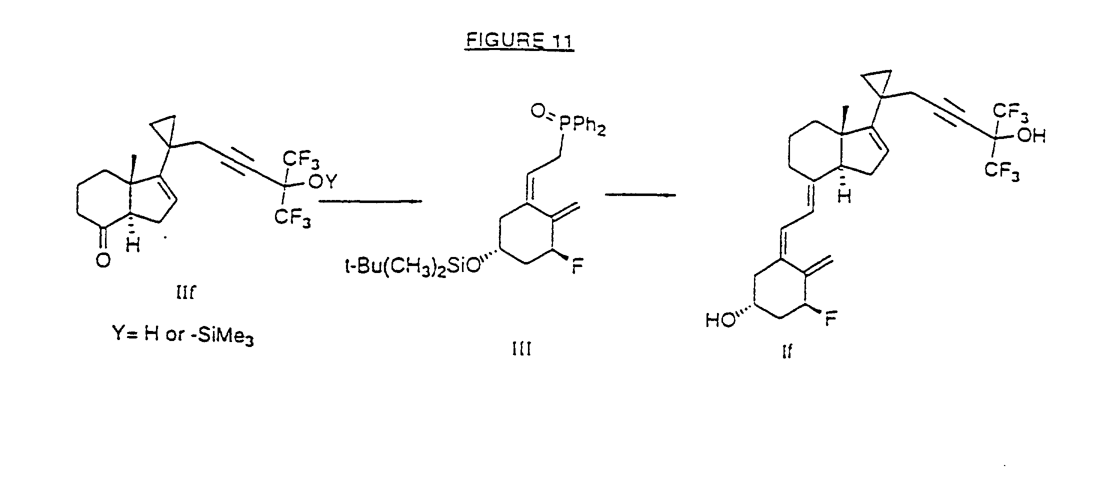

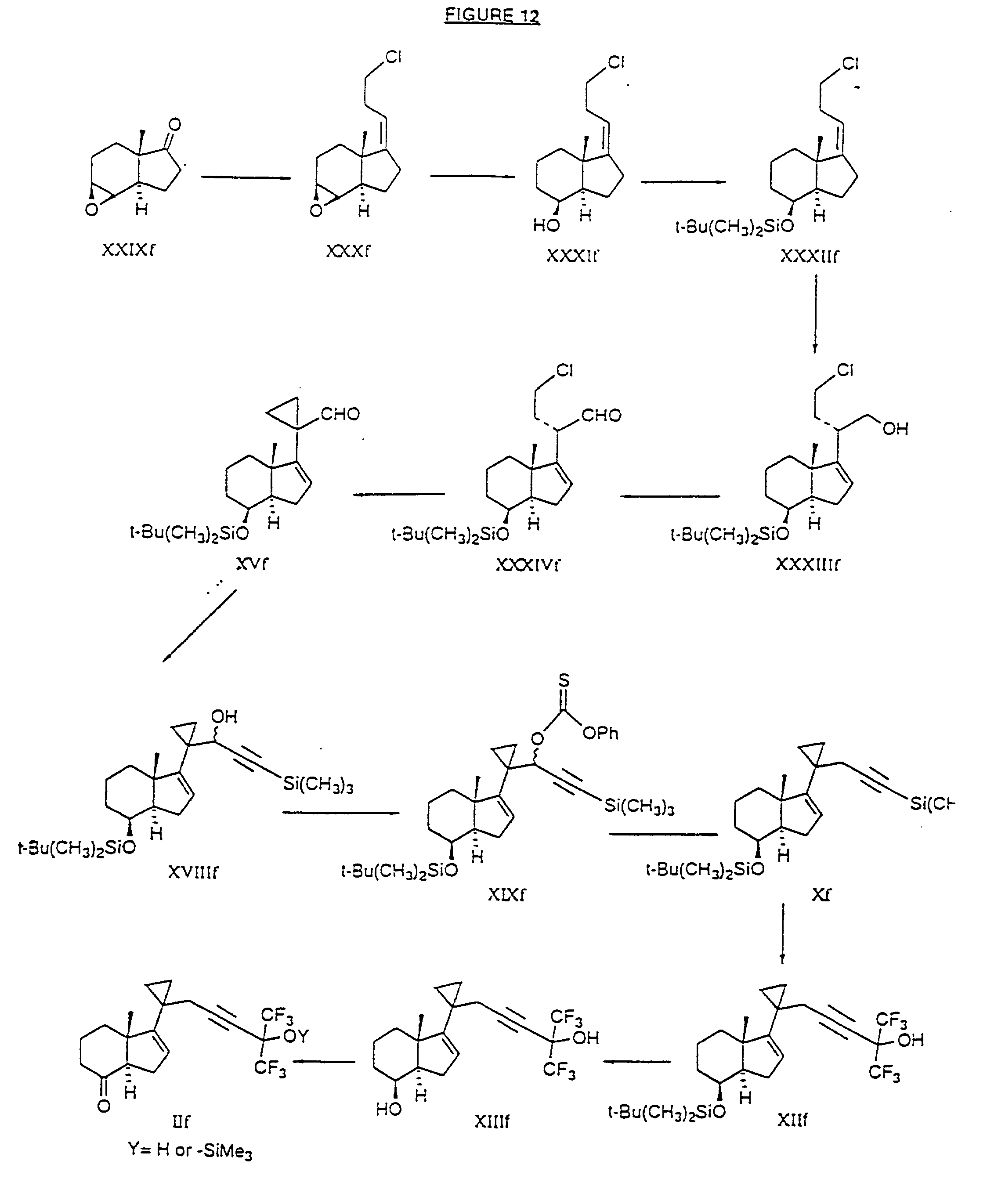

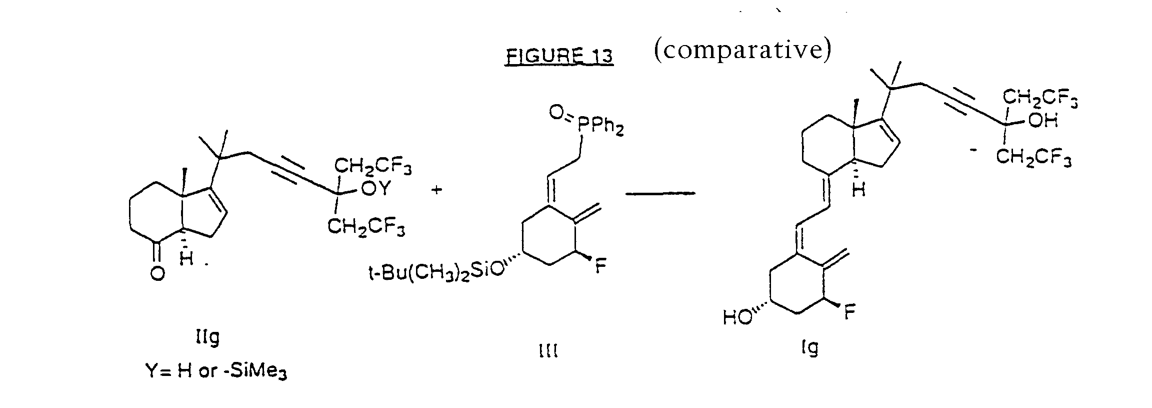

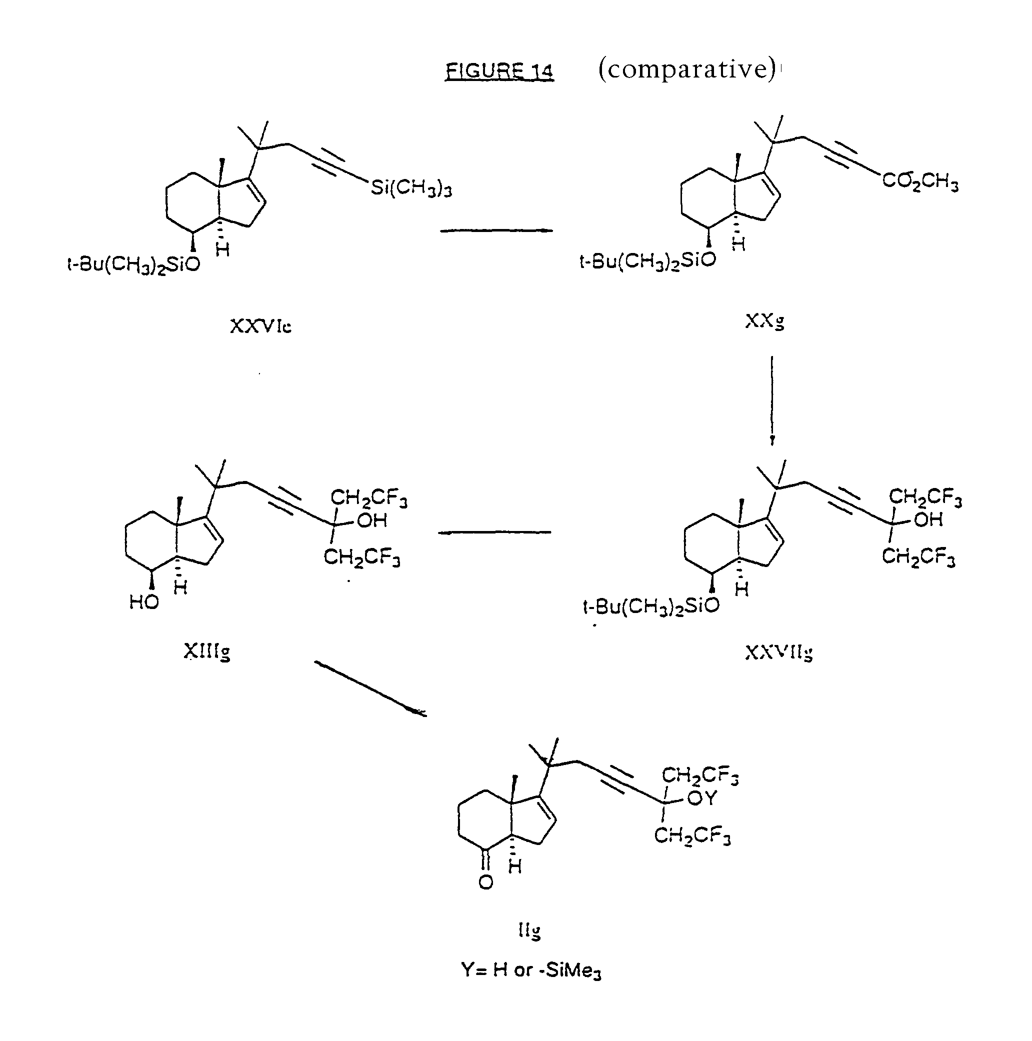

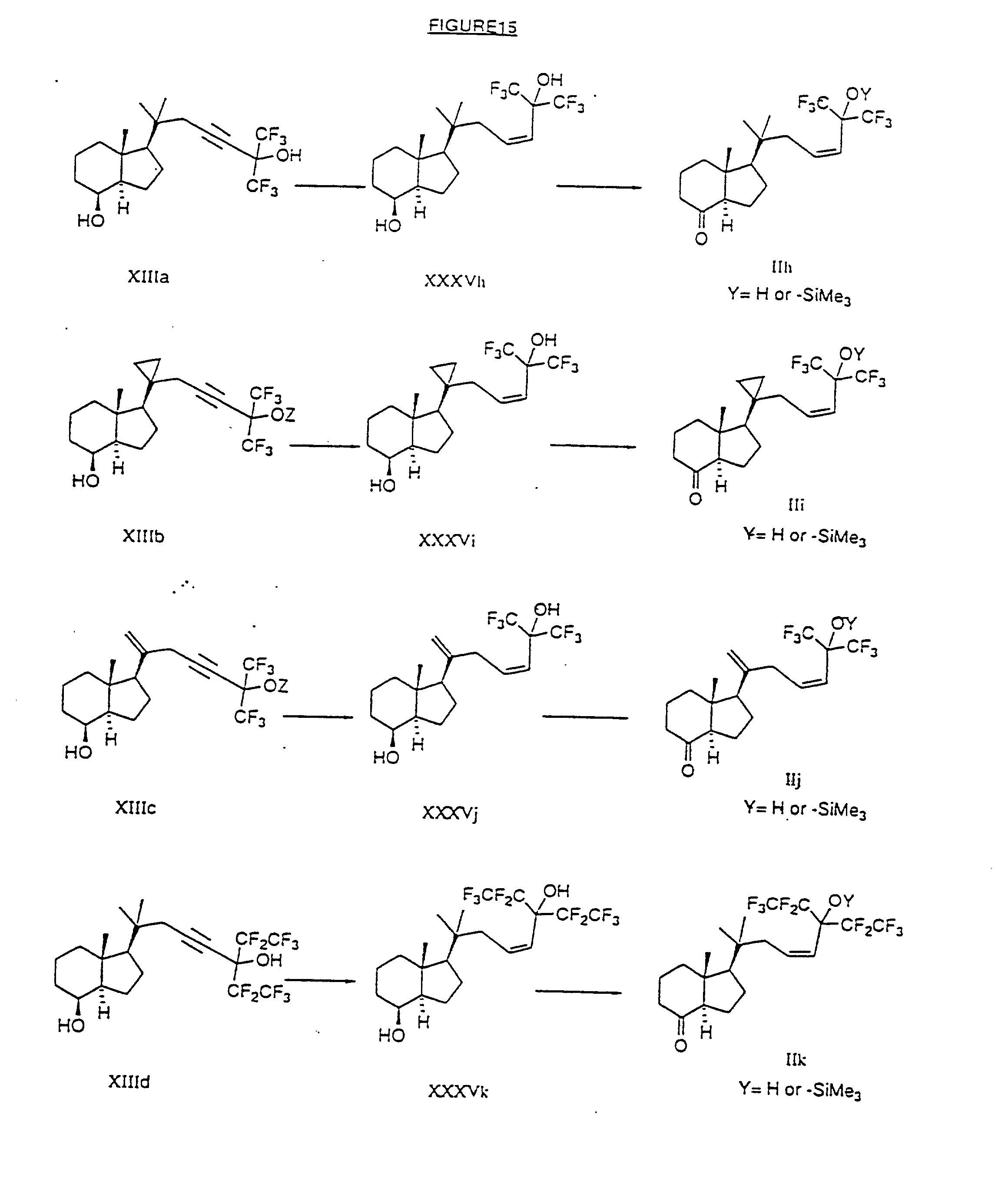

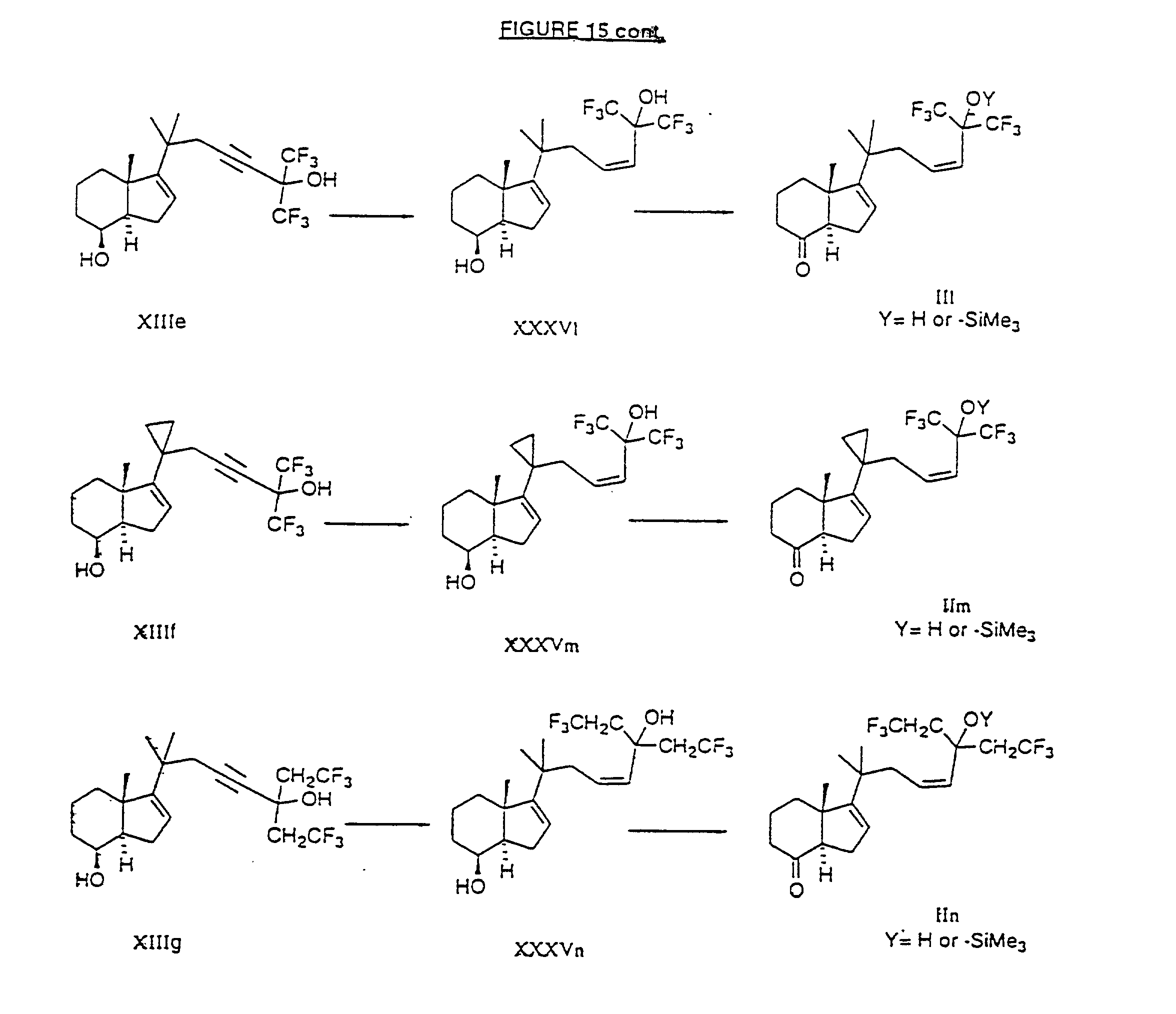

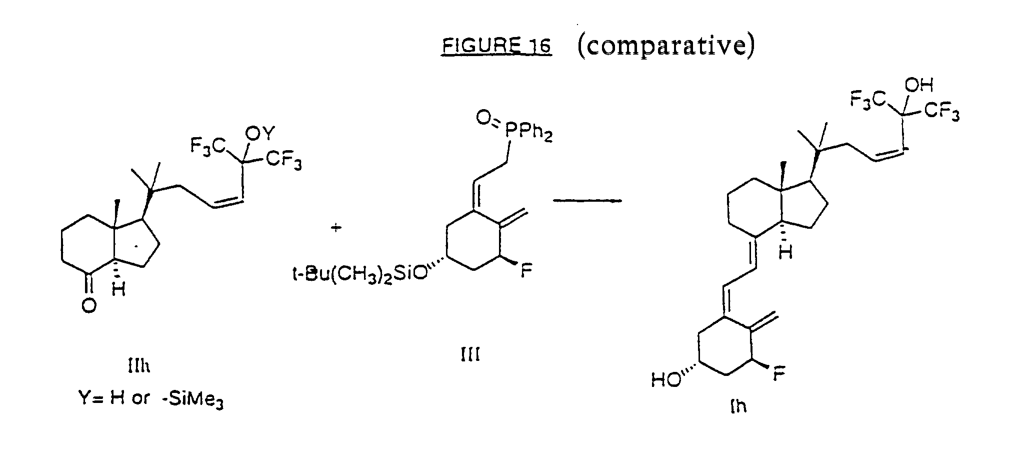

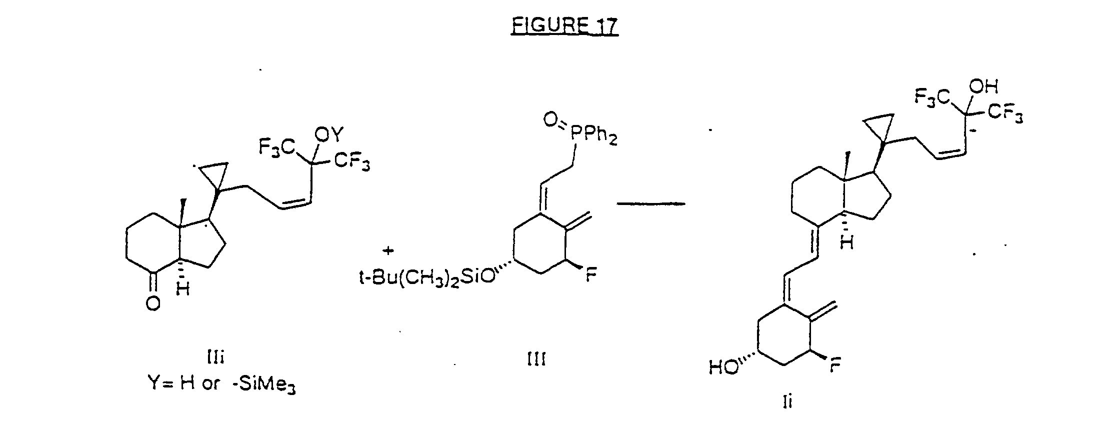

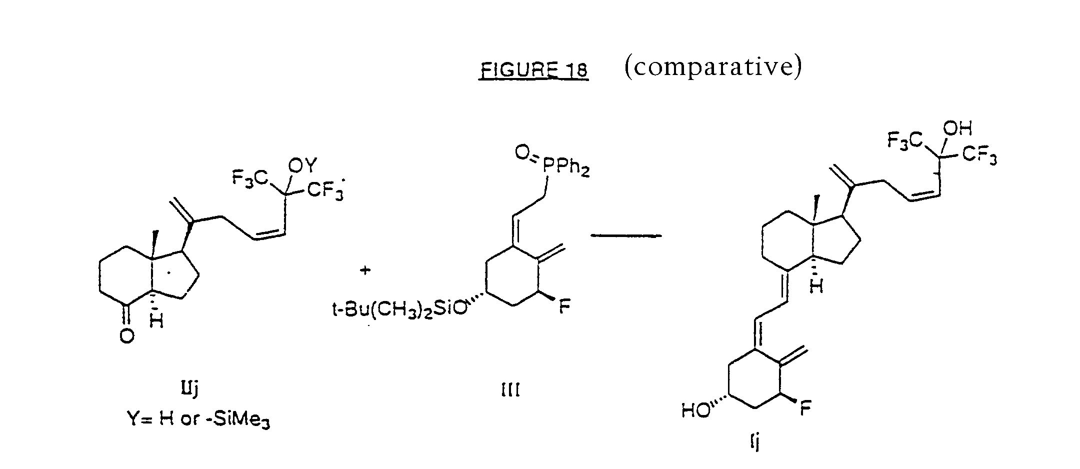

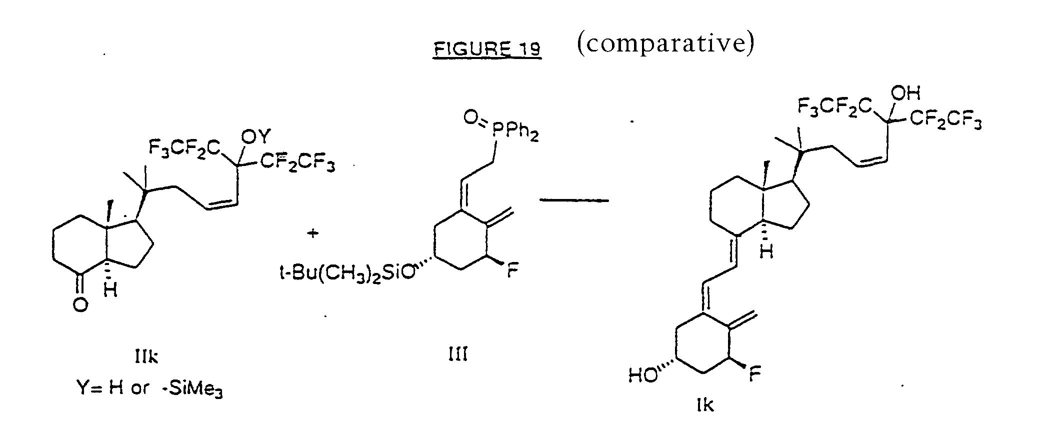

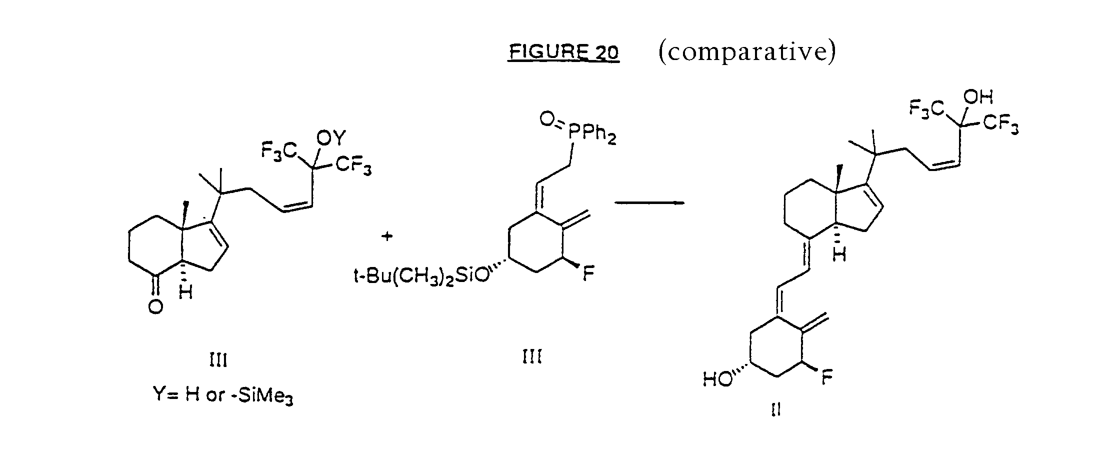

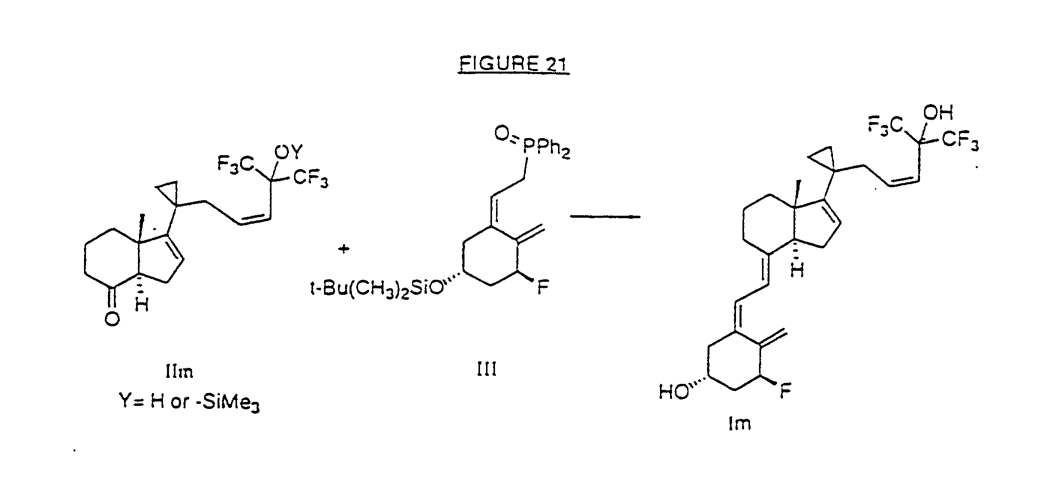

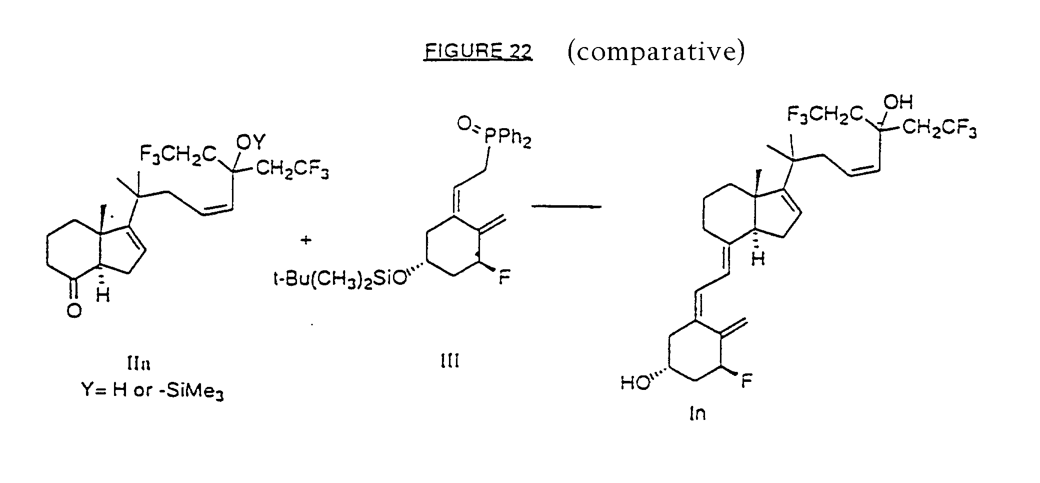

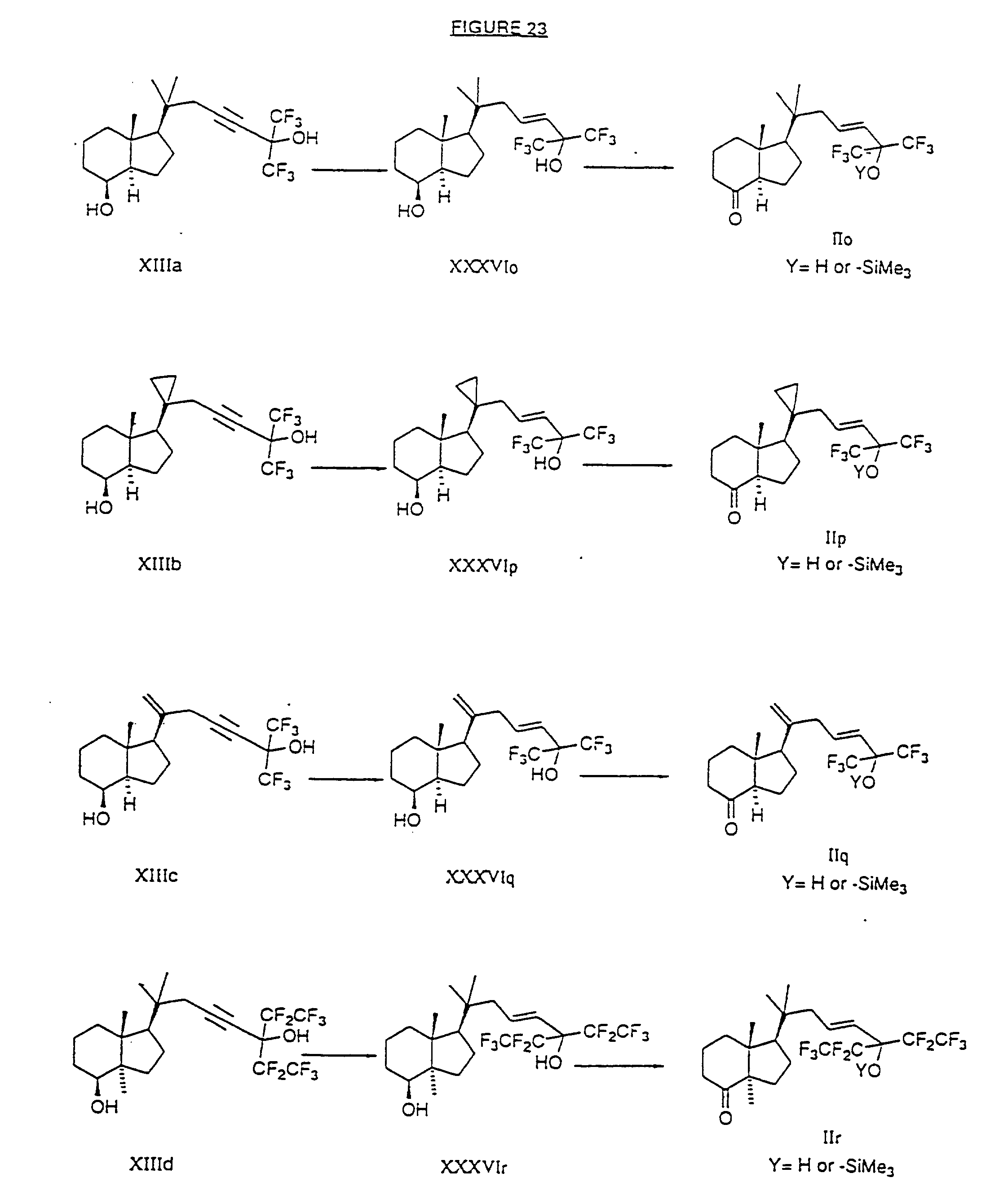

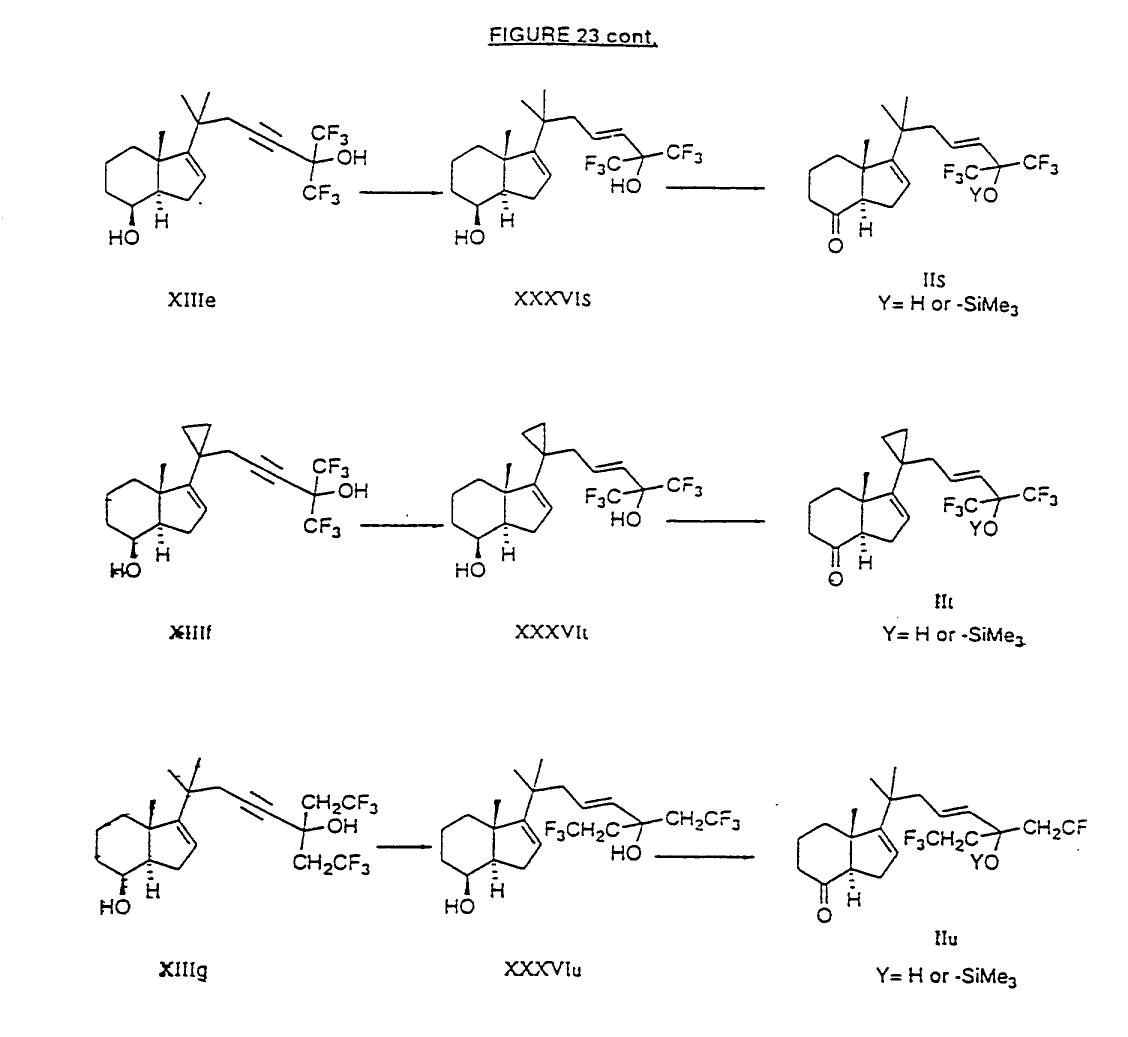

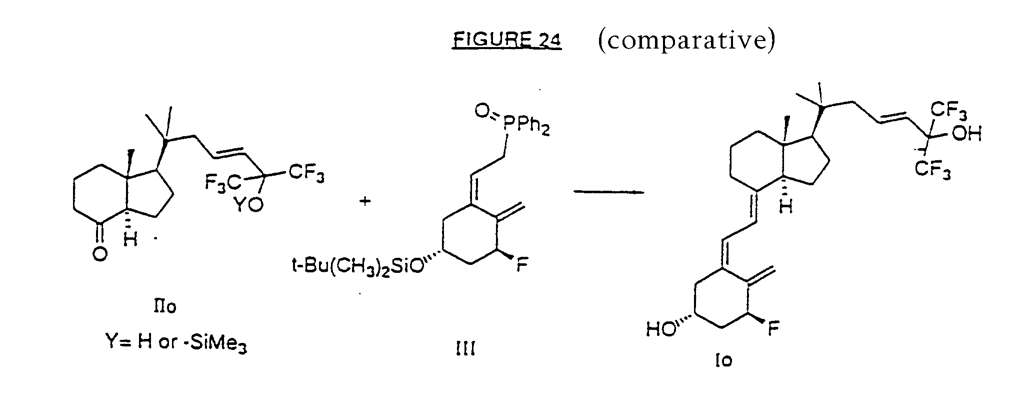

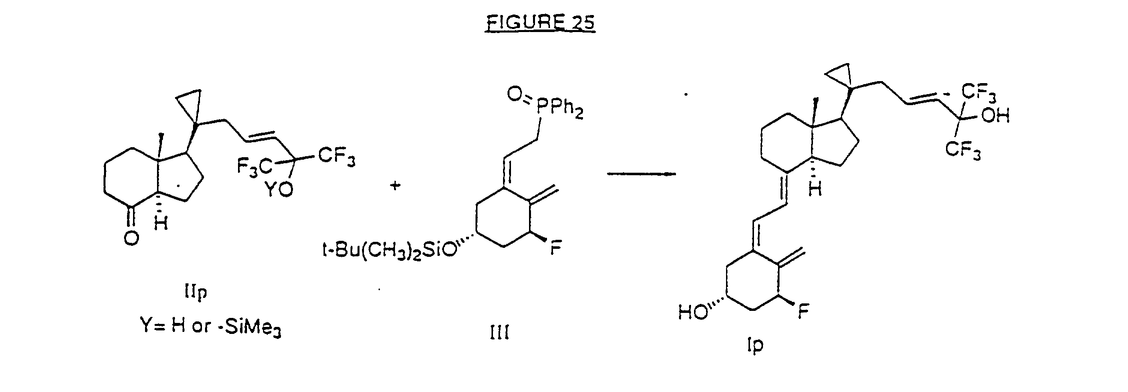

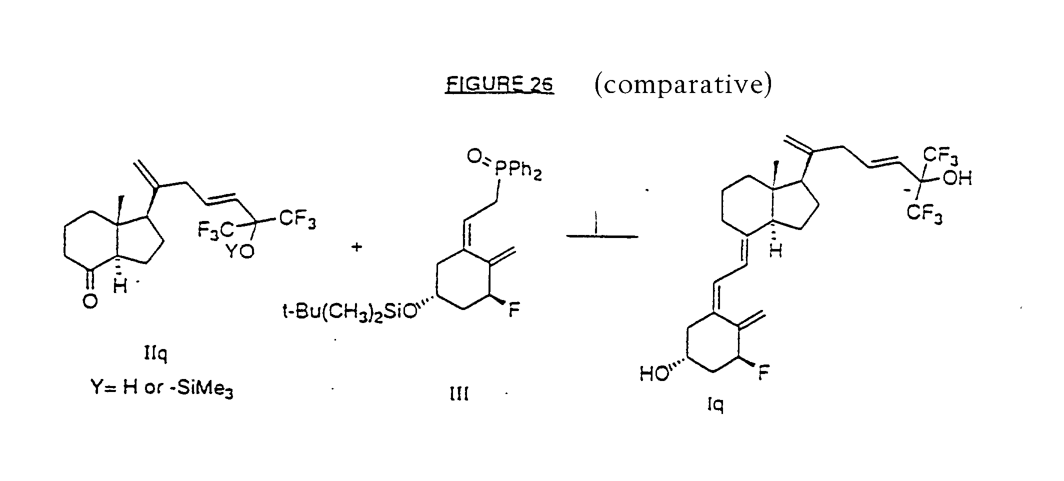

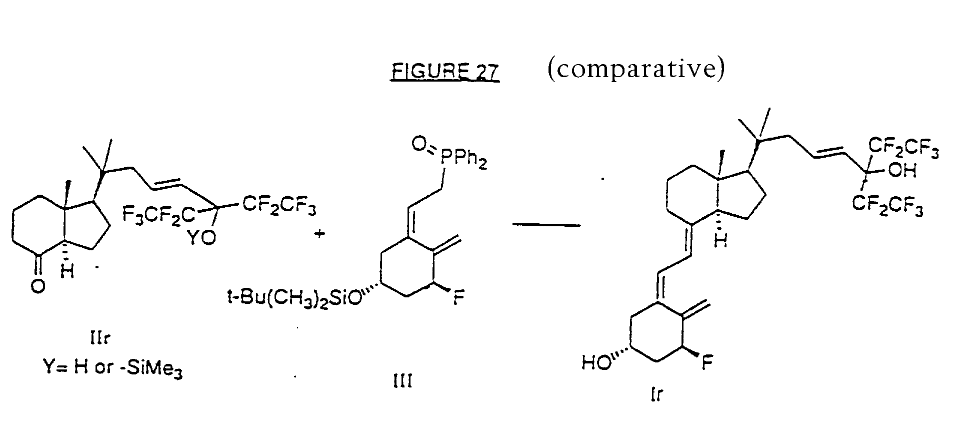

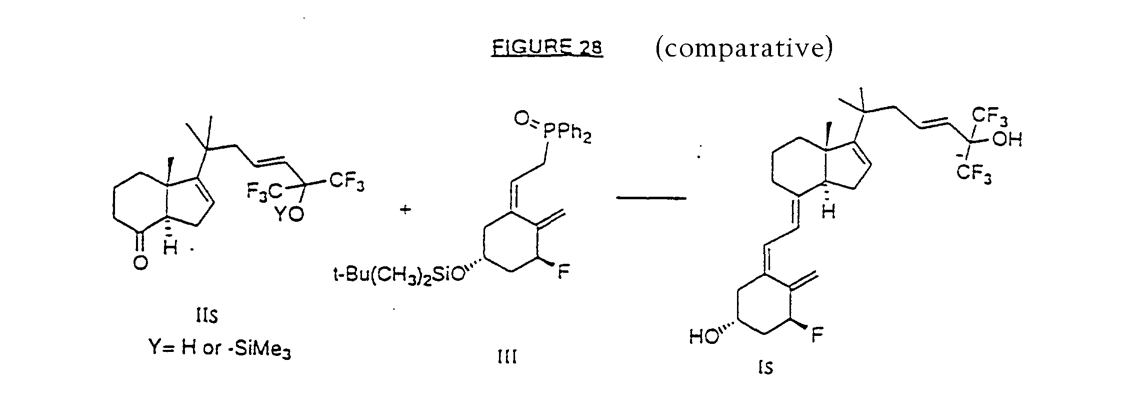

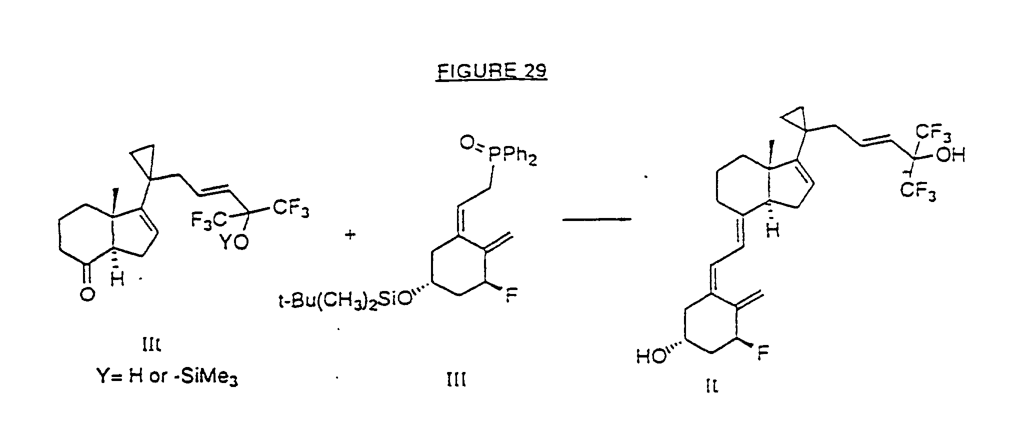

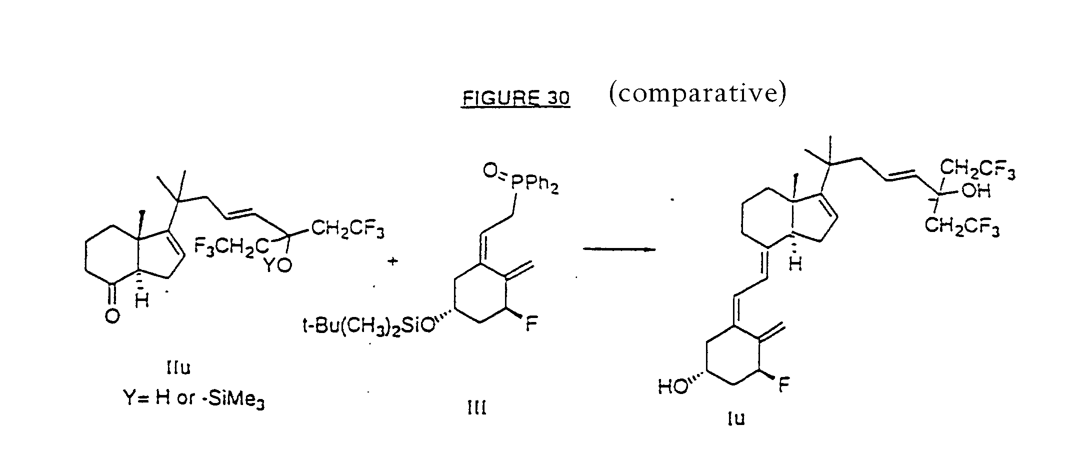

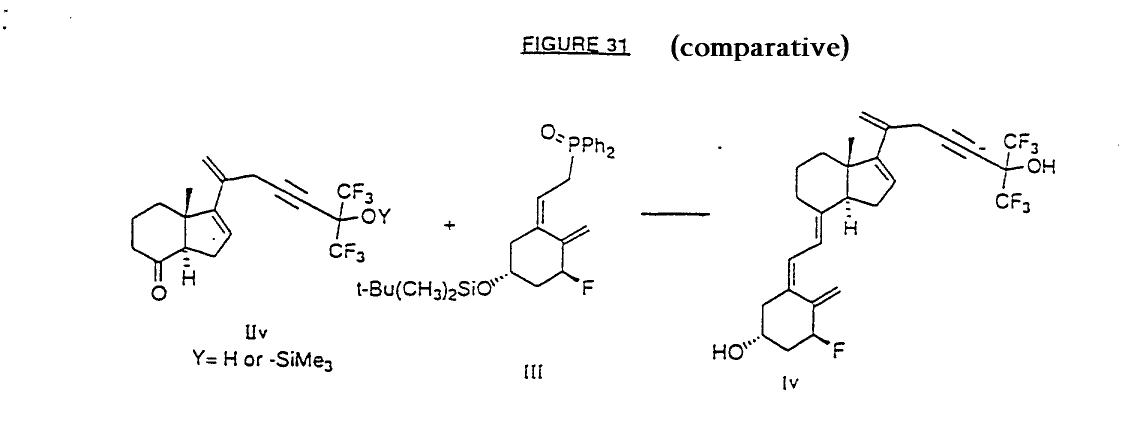

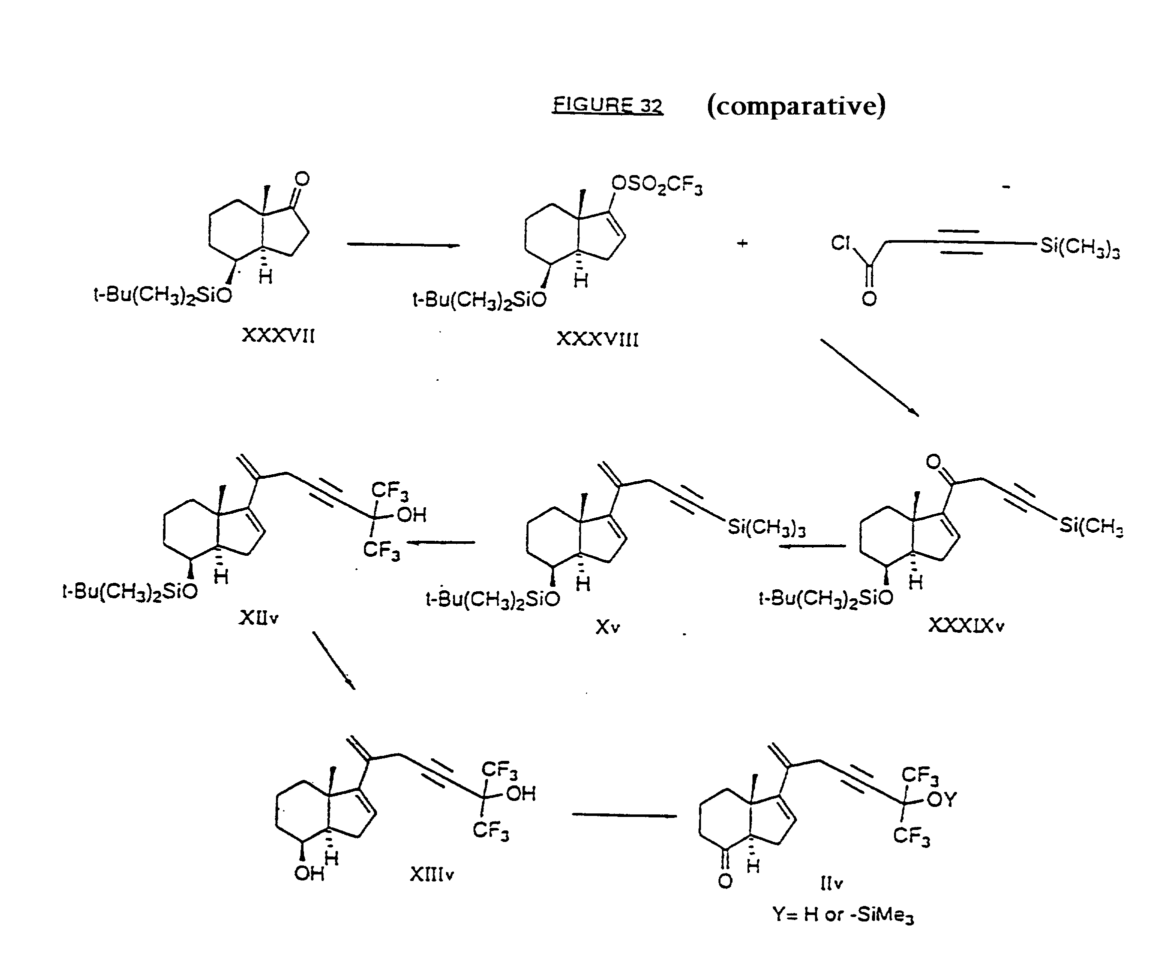

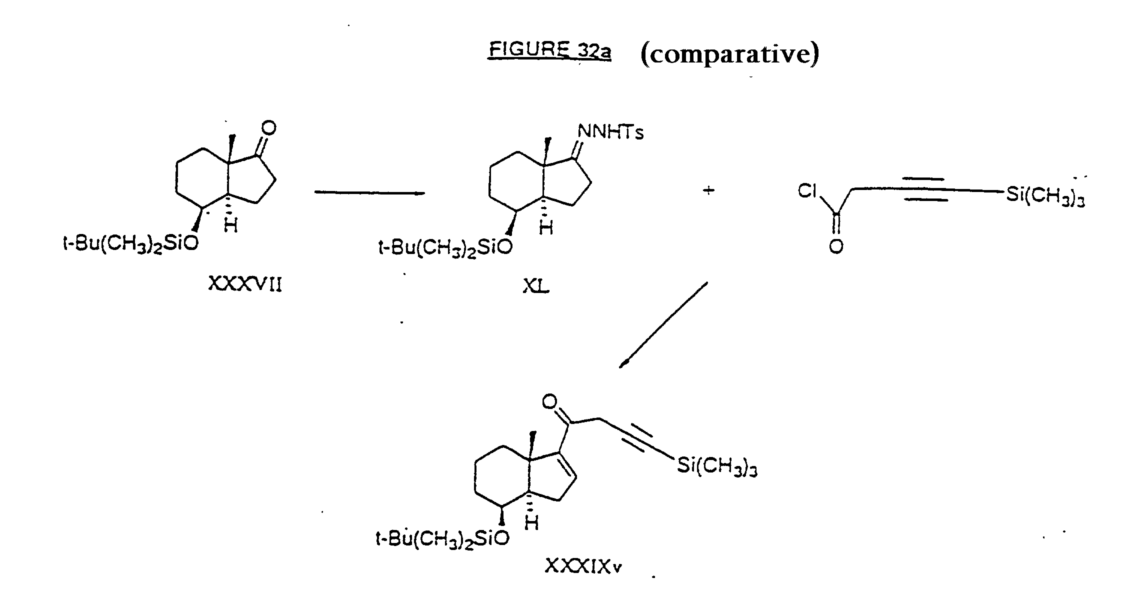

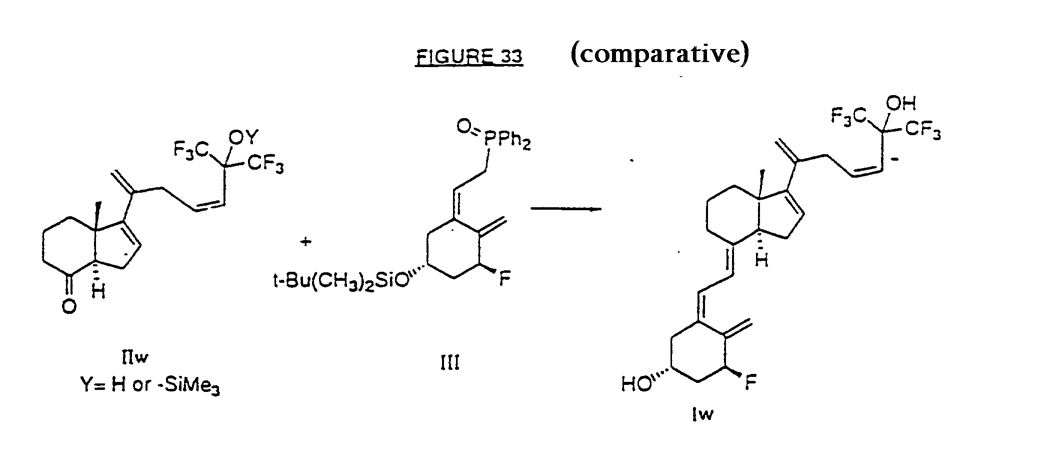

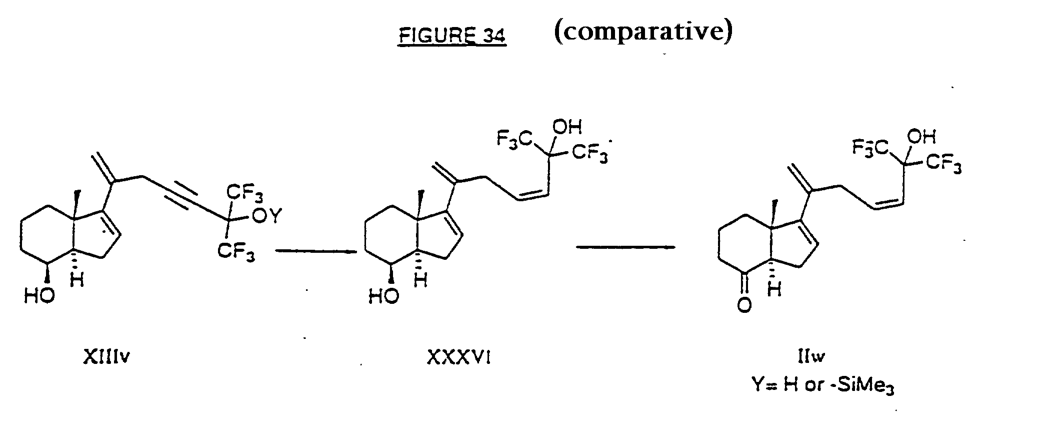

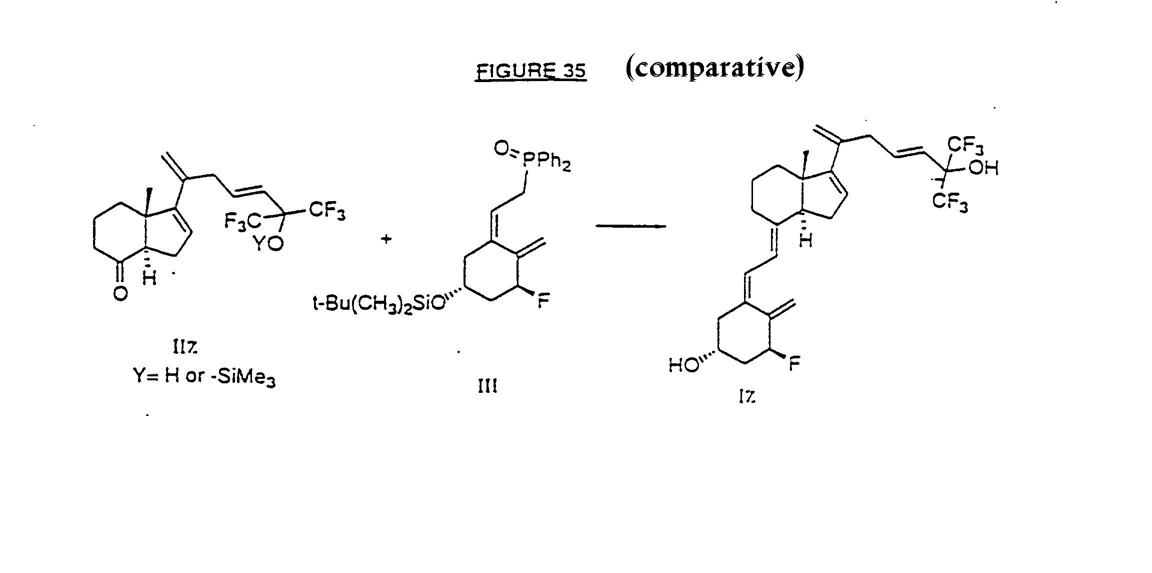

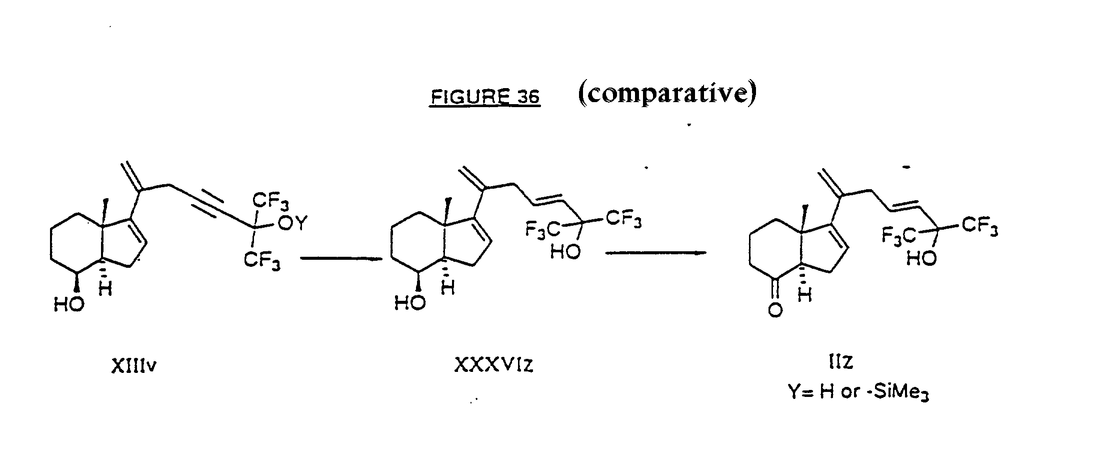

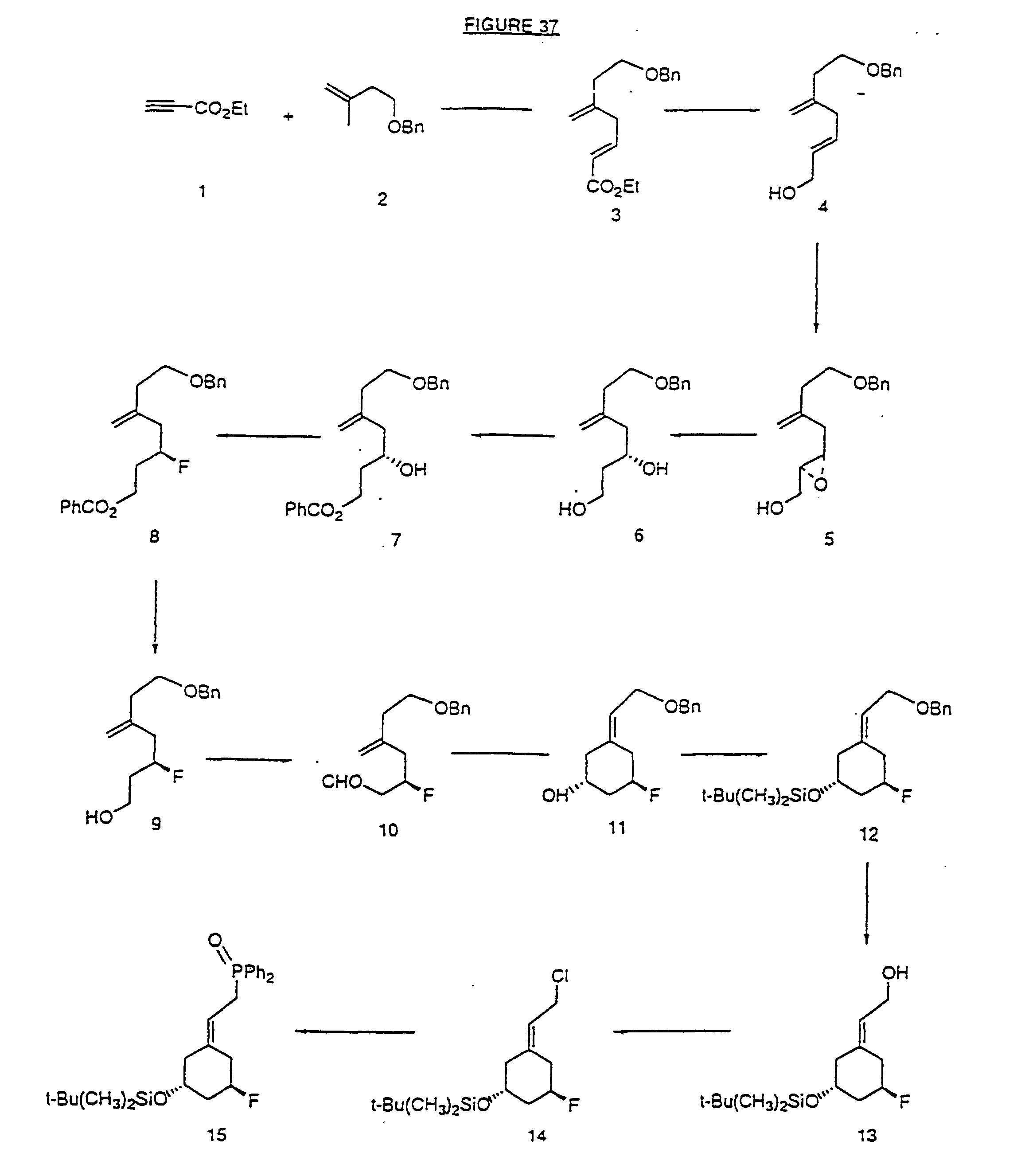

As used herein, the term (C₁-C₄) alkyl means a fully-saturated hydrocarbon radical having from one to four carbon atoms; a (C₁-C₄) fluoroalkyl is an alkyl radical, as defined above, in which one or more hydrogen atoms attached to the carbon backbone have been replaced with one or more fluorine atoms. A (C₃-C₆) cycloalkyl means a cyclic saturated hydrocarbon radical having from three to six carbon atoms, a (C₃-C₆) cyclofluoroalkyl means a cycloalkyl radical, as defined above, in which one or more hydrogen atoms attached to the carbon backbone have been replaced with one or more fluorine atoms. A (C₃-C₉) cycloalkyl is a cyclic saturated hydrocarbon radical having from three to nine carbon atoms; a (C₃-C₉) cyclofluoroalkyl is a cyclic saturated hydrocarbon radical having from three to nine carbon atoms in which one or more hydrogen atoms attached to the carbon backbone have been replaced with one or more fluorine atoms.

Also as used herein, cyclopropano means a cyclopropane radical; cyclodifluoropropano means a cyclopropane radical substituted with two fluorine atoms; cyclotetrafluoropropano means a cyclopropane radical substituted with four fluorine atoms; cyclopentano means a fully-saturated five-carbon ring radical; cyclodifluoropentano means a saturated five-carbon ring radical substituted with two fluorine atoms; cyclotetrafluoropentano means a saturated five-carbon ring radical substituted with four fluorine atoms; cyclohexafluoropentano means a saturated five-carbon ring radical substituted with six fluorine atoms; cycloctafluoropentano means a saturated five-carbon ring radical substituted with eight fluorine atoms; cyclohexano means a fully-saturated six-carbon ring radical; cyclodifluorohexano means a saturated six-carbon ring radical substituted with two fluorine atoms; cyclotetrafluorohexano means a saturated six-carbon ring radical substituted with four fluorine atoms; cyclohexafluorohexano means a saturated six-carbon ring radical substituted with six fluorine atoms; cycloctafluorohexano means a saturated six-carbon ring radical substituted with eight fluorine atoms.

Further as used herein, by double bond it is meant an unsaturated linkage between two adjacent carbon atoms in which two pairs of electrons are shared equally, and wherein each carbon atom bears two single-bonded substituents in either a Z (zusammen) or an E (entgegen) configuration about the double bond.

The compounds disclosed herein have not previously been described, nor has their use in the treatment of osteoporosis been recognized.

The compounds of the present invention may be generically described as 1α-fluoro analogs of vitamin D₃ (cholecalciferol). Hence, the compounds of the invention are named using the vitamin D₃ numbering system as illustrated in Figure (A) below.

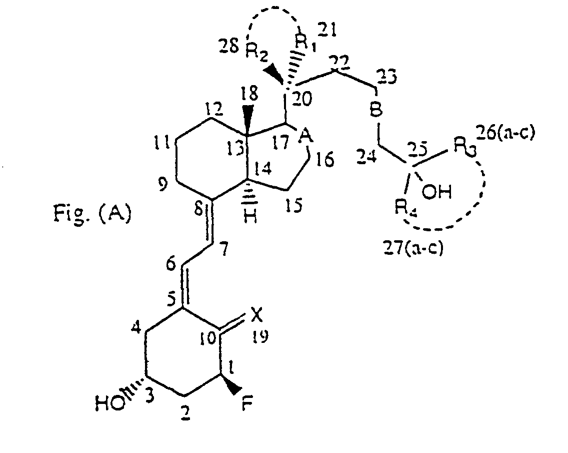

For example, a compound where R₁ is hydrogen, R₂ is CH₃, A is a double bond, B is a triple bond and R₃ is CF₃ is named as 1α-fluoro-25-(RS)-hydroxy-16-ene-23-yne- 26-trifluoro-20-epi-cholecalciferol.

The following table provides some representative examples of compounds of the present invention:
(Compounds not falling within the ambit of the present invention are marked "*")

| Cpd # | A | B | R₁ | R₂ | R₃ | R₄ | X |
|---|---|---|---|---|---|---|---|
| 1* | = | ≡ | CH₃ | CH₃ | CH₂CH₃ | CH₂CH₃ | =CH₂ |
| 2 * | = | ≡ | H | CH₃ | CH₂CH₃ | CH₂CH₃ | H |
| 3 * | = | ≡ | CH₃ | H | CH₂CH₃ | CH₂CH₃ | H |
| 4 | = | ≡ | CH₂-CH₂ | | CH₂CH₃ | CH₂CH₃ | =CH₂ |
| 5 | = | ≡ | CF₂-CF₂ | | CH₂CH₃ | CH₂CH₃ | =CH₂ |
| 6 | = | ≡ | CF₂-CH₂ | | CH₂CH₃ | CH₂CH₃ | =CH₂ |
| 7 | = | ≡ | CH₂-CF₂ | | CH₂CH₃ | CH₂CH₃ | =CH₂ |
| 8 * | = | ≡ | CF₃ | CF₃ | CH₂CH₃ | CH₂CH₃ | =CH₂ |
| 9 * | = | ≡ | H | CF₃ | CH₂CH₃ | CH₂CH₃ | =CH₂ |
| 10 * | = | ≡ | CF₃ | H | CH₂CH₃ | CH₂CH₃ | =CH₂ |
| 11 * | = | ≡ | CH₃ | CH₃ | CH₃ | CH₃ | =CH₂ |
| 12 * | = | ≡ | CH₃ | CH₃ | CF₃ | CF₃ | =CH₂ |
| 13 * | = | ≡ | CH₃ | CH₃ | CH₂CF₃ | CH₂CF₃ | =CH₂ |
| 14 * | = | ≡ | CH₃ | CH₃ | CF₂CH₃ | CF₂CH₃ | =CH₂ |
| 15 * | = | ≡ | CH₃ | CH₃ | CF₂CF₃ | CF₂CH₃ | =CH₂ |
| 16 * | - | ≡ | CH₃ | CH₃ | CH₂CH₃ | CH₂CH₃ | =CH₂ |
| 17 | - | ≡ | CH₂-CF₂ | | CF₂CH₃ | CF₂CH₃ | =CH₂ |
| 18 * | - | ≡ | H | CF₃ | CF₂CF₃ | CF₂CF₃ | =CH₂ |
| 19 * | = | trans C=C bond | CH₃ | CH₃ | CH₂CH₃ | CH₂CH₃ | =CH₂ |
| 20 | - | trans C=C bond | CH₂-CH₂ | | CF₃ | CF₃ | =CH₂ |
| 21 * | = | cis C=C bond | CH₃ | CH₃ | CH₂CF₃ | CH₂CF₃ | =CH₂ |
| 22 | - | cis C=C bond | CF₂-CH₂ | | CH₂CH₃ | CH₂CH₃ | =CH₂ |
| 23 * | = | ≡ | CH₃ | CH₃ | CH₂CH₃ | CH₂CH₃ | H |
| 24 * | - | trans C=C bond | H | CF₃ | CF₂CH₃ | CF₂CH₃ | H |
| 25 * | = | ≡ | CH₂ | | CH₂CH₃ | CH₂CH₃ | =CH₂ |
| 26 * | - | ≡ | CH₂ | | CF₃ | CF₃ | H |
| 27 * | = | ≡ | CH₃ | CH₃ | CH₂-CH₂ | | =CH₂ |
| 28 * | = | ≡ | CF₃ | H | CF₂-CF₂ | | =CH₂ |
| 29 * | = | ≡ | CF₂-CF₂ | | CF₂-CF₂ | | =CH₂ |
| 30 * | = | ≡ | CH₃ | CH₃ | CF₂CF₂-CF₂CF₂ | | =CH₂ |
| 31 * | = | ≡ | CH₂ | | CF₂CF₂-CF₂CF₂ | | =CH₂ |
| 32 * | - | cis C=C bond | CH₂ | | CH₂CF₂-CH₂CF₂ | | H |
| 33 * | = | ≡ | CF₃ | CF₃ | CF₃ | CF₃ | =CH₂ |
| 34 * | = | ≡ | CH₃ | CF₃ | CH₃ | CH₃ | =CH₂ |
| 35 * | - | ≡ | H | CH₃ | CH₃ | CF₃ | =CH₂ |
| 36 * | - | ≡ | CH₃ | CH₃ | CF₃ | CF₃ | =CH₂ |
| 37 * | - | cis C=C bond | CH₃ | CH₃ | CF₃ | CF₃ | =CH₂ |
| 38 * | = | trans C=C bond | CH₃ | CH₃ | CH₂CF₃ | CH₂CF₃ | =CH₂ |
| 39 * | - | ≡ | CH₃ | CH₃ | CF₃ | CF₃ | H |
| 40 | - | ≡ | CH₂-CH₂ | | CF₃ | CF₃ | =CH₂ |
| 41 * | = | ≡ | CH₂-CH₂ | | CH₂CH₂-CH₂CH₂ | | =CH₂ |
| 42 * | = | ≡ | CH₂-CF₂ | | CF₂CF₂-CF₂CF₂ | | =CH₂ |
| 43 * | - | trans C=C bond | CH₂ | | CH₂CH₂-CH₂CH₂ | | =CH₂ |
| 44 * | - | cis C=C bond | CF₂-CF₂ | | CH₂-CH₂ | | H |
| 45 * | - | ≡ | CH₂ | | CH₂CH₂-CH₂CH₂ | | =CH₂ |
| 46 * | = | ≡ | CH₂ | | CF₂-CF₂ | | =CH₂ |
| 47 * | = | trans C=C bond | CH₂-CH₂ | | CH₂-CH₂ | | H |
| 48 * | = | ≡ | CH₂-CF₂ | | CF₂-CF₂ | | =CH₂ |
| 49 * | - | ≡ | CH₃ | CH₃ | CH₃ | CF₃ | =CH₂ |
| 50 * | = | ≡ | H | CH₃ | CH₂CH₃ | CH₃ | H |
| 51 * | = | ≡ | CH₃ | CF₃ | CF₃ | CH₂CF₃ | =CH₂ |
| 52 * | - | ≡ | CF₃ | CF₃ | CH₃ | CF₂CF₃ | H |
| 53 | - | trans C=C bond | CH₂-CH₂ | | CF₂CF₃ | CF₃ | =CH₂ |
| 54 | = | cis C=C bond | CH₂-CF₂ | | CF₃ | CH₂CH₃ | H |
| 55 * | = | ≡ | CH₂ | | CF₃ | CH₃ | H |
| 56 * | = | ≡ | H | CH₃ | CF₃ | CH₃ | =CH₂ |
| 57 * | = | trans C=C bond | H | CH₃ | CF₃ | CH₃ | =CH₂ |
| 58 | - | ≡ | CH₂-CH₂ | | CH₃ | CH₃ | =CH₂ |
| 59 * | = | ≡ | H | CH₃ | CH₂CH₂-CH₂CH₂ | | =CH₂ |
| 60 | - | trans = bond | CH₂-CH₂ | | CF₃ | CF₃ | =CH₂ |
| 61 | - | = | CH₂-CH₂ | | CF₃ | CF₃ | =CH₂ |

and are named as:
56. 1-α-Fluoro-25(RS)-hydroxy-16-ene-23-yne-26-trifluoro-20-epi-cholecalciferol.
57. 1-α-Fluoro-25(RS)-hydroxy-16,23E-diene-26-trifluoro-20-epi-cholecalciferol. [α]_{D}²⁵ = +75.5° (c 0.2, EtOH); UV (MeOH): λmax 206nm (ε 17325), 243 (13485), 268 (13189).
58. 1-α-Fluoro-25-hydroxy-23-yne-20,21,28-cyclopropylcholecalciferol.
59. 1-α-Fluoro-25-hydroxy-16-ene-23-yne-25,26,27-cyclopentyl-20-epi-cholecalciferol. [α]_{D}²⁵ = +60.5° (c 0.2, CHCl₃).
60. 1-α-Fluoro-25-hydroxy-23E-ene-26,27-hexafluoro-20,21,28-cyclopropyl-cholecalciferol.
61. 1-α-Fluoro-25-hydroxy-23-yne-26,27-hexafluoro-20,21,28-cyclopropyl-cholecalciferol.

### General Synthesis

Analogs of this invention may generally be prepared by reaction and combination of fragments of Vitamin D₃ molecules (*see e.g.*, Shiuey et al., *J. Org. Chem,* 55:243 (1990); Wovkulich, P.M. et al., *Tetrahedron,* 40, 2283 (1984); Doran et al., U.S. Patent No. 5,428,029; and Baggiolini et al., U.S. Patent No. 5,451,574). The starting materials and reagents used in preparing these compounds are either available from commercial suppliers such as Aldrich Chemical Co., (Milwaukee, WI), or Sigma (St. Louis, MO) or they can be prepared by methods known to those skilled in the art following procedures set forth in references such as Fieser and Fieser's *Reagents for Organic Synthesis*, Vol. 1-15 (John Wiley and Sons, 1991); March's *Advanced Organic Chemistry,* (John Wiley and Sons 4th Edition) and Larock's *Comprehensive Organic Transformations* (VCH Publishers Inc., 1989).
The preparation of compounds of Formula I and the intermediates used in their preparation is illustrated by the reaction schemes as shown in the Drawings.

### 23-Yne Analogs

a. Synthesis of the 20-methyl analog (Ia) of 1α-fluoro-25-hydroxy-23-yne-26,27-hexafluoro-cholecalciferol is illustrated in Figure 1, and described in Example 1, below.
   The key intermediate IIa where Y is hydrogen or -SiMe₃ can be prepared according to the synthesis described in Figure 2, starting from the known compound IVa. Dehydration of IVa by known methods produces the ene product Va, which can be selectively oxidized with selenium dioxide to the allylic alcohol VIa. Cyclopropanation by standard methods gives the cyclopropyl alcohol VIIa, which on hydrogenation produces the desired dimethyl intermediate VIIIa. Extension of the side chain with the acetylene fragment can be performed by a previously known procedure consisting of displacement of the tosyloxy group in IXa with lithium trimethylsilyl acetylide. The completion of the side chain can be accomplished by treatment of the lithium acetylide derived from Xa or XIa with hexafluoroacetone. Removal of the silyl protective group and oxidation completes the synthesis of the desired compound IIa where Y is hydrogen. Compound IIa where Y is hydrogen can be converted to the corresponding trimethylsilyl ether derivative (Y = -SiMe₃), if desired, by reacting it with a suitable silylating agent such as 1-trimethylsilylimidazole in an aprotic organic solvent such as methylene chloride.
   The intermediate VIIIa in the synthesis of the compound IIa (Figure 2) can also be obtained from the same starting material IVa using an alternative process described in Figure 2a. The aldehyde XIV is methylated by standard methods, and thus obtained dimethyl aldehyde XVa is reduced to VIIIa.
b. Synthesis of the 20-cyclopropyl analog (Ib) of 1α-fluoro-25-hydroxy-23-yne-26,27-hexafluoro-cholecalciferol is illustrated in Figure 3 and described in Example 2.
   The key intermediate IIb where Y is hydrogen or -SiMe₃ can be obtained from the cyclopropyl alcohol VIIa, already described in Figure 2. This synthesis of IIb is shown in Figure 4, and it is in accordance with the process shown in Figure 2 for the synthesis of IIa. Anal. data for IIb (Y = hydrogen): mp 145-146°C; [α]_{D}²⁵ = -8.52° (c 0.704, EtOH); MS m/z 396.
   An alternative pathway to the intermediate Xb in Figure 4 is also possible. It consists of using the allylic alcohol VIa (Figure 2) as a starting material and it is illustrated in Figure 4a. Oxidation by standard methods gives the unsaturated aldehyde XVIb, which upon treatment with lithium trimethylsilyl acetylide gives the epimeric allylic propargylic alcohols XVIIb. Cyclopropanation will lead selectively to the cyclopropyl alcohols XVIIIb, which on Barton deoxygenation gives the desired Xb, precursor of IIb.
c. Synthesis of the 20-ene analog (Ic) of 1α-fluoro-25-hydroxy-23-yne-26,27-hexafluoro-cholecalciferol is illustrated in Figure 5, and described in Example 3.
   In this case, the key intermediate IIc where Y is hydrogen or -SiMe₃ can be synthesized from the epimeric allylic-propargylic alcohols XVIIb (Figure 4a) according to the process outlined in Figure 6. Barton deoxygenation produces in this case the ene-yne feature of the desired side chain, compound Xc. The lithium acetylide derived from Xc or XIc reacts with hexafluoroacetone to complete the side chain as in XIIc. Removal of the silyl protecting group and oxidation produces the desired ketone IIc (Y = hydrogen) which can be converted to the corresponding trimethylsilyl ether derivative (Y = -SiMe₃), if desired, as described above.
   The ene-yne intermediate XIc (Figure 6) can be also obtained from the early ene-precursor Va (Figure 2) as shown in Figure 6a. An ene-reaction with formaldehyde produces the C-23 homoallylic alcohol, from which the acetylene XIc can be constructed in two steps by known methods.
d. Synthesis of the 20-methyl-26,27-bishomo-26,26a,27,27a-decafluoro analog (Id) of 1α-fluoro-25-hydroxy-23-yne-cholecalciferol is illustrated in Figure 7, and described in Example 4.
   The key intermediate decafluoro-hydroxy-ketone IId where Y is hydrogen or -SiMe₃ can be synthesized according to the process illustrated in Figure 8. This synthesis starts from the previously cited silylated 20-methyl-acetylene Xa (Figure 2), which is first extended to the carboxylic ester XXd. Reaction with pentafluoroethyl lithium produces the complete side chain, the tertiary alcohol XIId. Removal of the protecting silyl group and oxidation gives compound IId (Y = hydrogen) which can be converted to the corresponding trimethylsilyl ether derivative (Y = -SiMe₃), if desired, as described previously.

### 16-Ene-23-yne-Analogs

e. Synthesis of the 20-methyl analog (Ie) of 1α-fluoro-25-hydroxy-16-ene-23-yne-26,27-hexafluoro-cholecalciferol is illustrated in Figure 9, and described in Example 5.
   The key intermediate IIe where Y is hydrogen or -SiMe₃ can be synthesized as shown in Figure 10. This synthesis starts with the known monosilylated diol XXIe, which on oxidation produces the aldehyde XXIIe. Alkylation of this aldehyde forms the dimethyl analog XXIIIe, which upon treatment with lithium trimethylsilyl acetylide produces the epimeric propargylic alcohols XXIVe. Barton deoxygenation gives the trimethylsilyl acetylene XXVIe. Lithium acetylide derived from XXVIe in a reaction with hexafluoroacetone produces the complete side chain XXVIIe. Removal of the silyl protecting group and oxidation lead to the desired intermediate IIe (Y = hydrogen) which can be converted to the corresponding trimethylsilyl ether derivative (Y = -SiMe₃) as described previously.
f. Synthesis of the 20-cyclopropyl analog (If) of 1α-fluoro-25-hydroxy-16-ene-23-yne-26,27-hexafluorocholecalciferol is illustrated in Figure 11, and described in Example 6.
   The key intermediate IIf where Y is hydrogen or -SiMe₃ can be synthesized from the known epoxy-ketone XXIXf according the process shown in Figure 12. The attachment of the incipient three carbons of the side chain by a Wittig reaction, reduction of the epoxide, protection of thus formed hydroxy group as a silyl ether, insertion of the C-20 aldehyde via an ene reaction oxidation, and cyclization of the cyclopropyl ring gives a compound of formula XVf. Completion of the IIf synthesis is according to the process described in Figures 4a and 4 for the saturated analog IIb.
g. Synthesis of the 20-methyl-26,27-bishomo 26a,27a-hexafluoro analog (Ig) of 1α-fluoro-25-hydroxy-16-ene-23-yne-cholecalciferol is illustrated in Figure 13, and described in Example 7.
   The key intermediate IIg where Y is hydrogen or -SiMe₃ can be prepared from the previously cited compound XXVIe (Figure 10) according to the process outlined in Figure 14. Extension of the side chain of XXVIe gives the propargylic ester XXg, which is then converted to the tertiary alcohol XXVIIg in a reaction with trifluoroethyl lithium. Removal of the silyl protecting group and oxidation produces the ketone IIg (Y = hydrogen) which can be converted to the corresponding trimethylsilyl ether derivative (Y = -SiMe₃) as described above.

### 23Z-Ene Analogs

For the synthesis of 23Z-ene vitamin D analogs with various modifications at C-20, the previously cited 23-yne intermediates XIII(a-g) may be hydrogenated with Lindlar catalyst to produce 23Z-ene congeners XXXV(h-m), from which the corresponding ketones II(h-m) where Y is hydrogen are obtained by oxidation as shown in figure 15. Ketones II (h-m) where Y is hydrogen can be converted to the corresponding trimethylsilyl ether derivatives, if desired, as described previously.
h. Synthesis of the 20-methyl analog (Ih) of 1α-fluoro-25-hydroxy-23Z-ene-26,27-hexafluoro-cholecalciferol is illustrated in Figure 16, and described in Example 8.
i. Synthesis of the 20-cyclopropyl analog (Ii) of 1α-fluoro-25-hydroxy-23Z-ene-26,27-hexafluoro-cholecalciferol is illustrated in Figure 17, and described in Example 9.
j. Synthesis of the 20-ene-analog (Ij) of 1α-fluoro-25-hydroxy-23Z-ene-26,27-hexafluoro-cholecalciferol is illustrated in Figure 18, and described in Example 10.
k. Synthesis of the 20-methyl-26,27-bishomo-26,26a,27,27a-decafluoro analog (Ik) of 1α-fluoro-25-hydroxy-23Z-ene-cholecalciferol is illustrated in Figure 19, and described in Example 11.

### 16,23Z-Diene Analogs

l. Synthesis of the 20-methyl analog (Il) of 1α-fluoro-25-hydroxy-16,23Z-diene-26,27-hexafluoro-cholecalciferol is illustrated in Figure 20, and described in Example 12.
m. Synthesis of the 20-cyclopropyl analog (Im) of 1α-fluoro-25-hydroxy-16,23Z-diene-26,27-hexafluorocholecalciferol is illustrated in Figure 21, and described in Example 13.
n. Synthesis of the 20-methyl-26,27-bishomo-26a,27a-hexafluoro analog (In) of la-fluoro-25-hydroxy-16,23Z-diene-cholecalciferol is illustrated in Figure 22, and described in Example 14.

### 23E-ene Analogs

For the synthesis of 23E-ene vitamin D analogs with various modifications at C-20, the previously cited 23-yne intermediates XIII(a-g) are reduced with lithium aluminum hydride in the presence of sodium methoxide to produce 23E-ene congeners XXXVI(o-u), from which the corresponding ketones II(o-u) where Y is hydrogen are obtained by oxidation as shown in figure 23. Ketones II (h-m) where Y is hydrogen can be converted to the corresponding trimethylsilyl ether derivatives, if desired, as described above.
Anal. data for intermediate IIp (Y =hydrogen): mp 79-80°C; [α]_{D}²⁵ = +6.9° (c 1.00, EtOH); MS m/z 398 (M⁺, 20).
o. Synthesis of the 20-methyl analog (Io) of 1α-fluoro-25-hydroxy-23E-ene-26,27-hexafluoro-cholecalciferol is illustrated in Figure 24, and described in Example 15.
p. Synthesis of the 20-cyclopropyl analog (Ip) of 1α-fluoro-25-hydroxy-23E-ene-26,27-hexafluoro-cholecalciferol is illustrated in Figure 25, and described in Example 16.
q. Synthesis of the 20-ene analog (Iq) of 1α-fluoro-25-hydroxy-23E-ene-26,27 hexafluoro-cholecalciferol is illustrated in Figure 26, and described in Example 17.
r. Synthesis of the 20-methyl 26,27-bishomo-26,26a,27,27a-decafluoro analog (Ir) of 1α-fluoro-25-hydroxy-23E-ene-cholecalciferol is illustrated in Figure 27, and described in Example 18.

### 16,23E-Diene Analogs

s. Synthesis of the 20-methyl analog (Is) of 1α-fluoro-25-hydroxy-16,23E-diene-26,27-hexafluoro-cholecalciferol is illustrated in Figure 28, and described in Example 19.
t. Synthesis of the 20-cyclopropyl analog (It) of 1α-fluoro-25-hydroxy-16,23E-diene-26,27-hexafluorocholecalciferol is illustrated in Figure 29, and described in Example 20.
u. Synthesis of the 20-methyl-26,27-bishomo-26a,27a-hexafluoro analog (Iu) of 1α-fluoro-25-hydroxy-16,23E-diene-cholecalciferol is illustrated in Figure 30, and described in Example 21.

### 16,20-Diene-23-yne Analogs

v. Synthesis of the 20-ene analog (Iv) of 1α-fluoro-25-hydroxy-16-ene-23-yne-26,27-hexafluoro-cholecalciferol is illustrated in Figure 31, and described in Example 22.
   The key intermediate IIv where Y is hydrogen or -SiMe₃ can be prepared according to the synthesis outlined in Figure 32. This synthesis starts from the known ketone XXXVII and constitutes a novel entry into the 16-ene class of vitamin D analogs. The side chain is attached by a palladium catalyzed coupling of the enol triflate XXXVIII with the trimethylsilylbutynoic acid chloride to give the ketone XXXIXv, from which the side chain of IIv is completed by standard methods. An alternative for the conversion of XXXVII to the ketone is the use of Shapiro reaction shown in Figure 32a.

### 16,20,23Z-Triene Analogs

w. Synthesis of the 20-ene analog (Iw) of 1α-fluoro-25-hydroxy-16,23Z-diene-26,27-hexafluoro-cholecalciferol is illustrated in Figure 33, and described in Example 23. The key intermediate IIw where Y is hydrogen or -SiMe₃ can be prepared according to Figure 34 starting from the previously cited compound XIIIv (Figure 32) by hydrogenation using the Lindlar catalyst, followed by oxidation and silylation.

### 16,20,23E-Triene Analogs

z. Synthesis of the 20-ene analog (Iz) of 1α-fluoro-25-hydroxy-16,23E-diene-26,27-hexafluoro-cholecalciferol is illustrated in Figure 35, and described in Example 24. In this case the key intermediate IIz where Y is hydrogen can be prepared according to Figure 36 starting from XIIIv (Scheme 32) by reduction with lithium aluminum hydride in the presence of sodium methoxide, followed by oxidation. IIz where Y is hydrogen can be converted to the corresponding trimethylsilyl ether derivative, if desired, as described previously.

### 1a-Fluoro-19-Nor Analogs

The precursor (3R-(3α,5β,Z)]-2 [2-[3-fluoro-5-[[(1,1-dimethylethyl)dimethylsilyl]oxy]cyclo-hexylidene]ethyl] diphenyl phosphine oxide (**15**) needed for the synthesis of 1α-fluoro-19-nor Vitamin D analogs can be prepared by the synthesis outlined as follows and illustrated in Figure 37. Synthesis of a specific 1α-fluoro-19-nor analog is described in Example 25.
The diene-ester (**3**) is obtainable by an ene reaction starting from ethyl propionate (**1**) and 3-methyl-but-3-en-1-ol benzyl ether (**2**) catalyzed with ethyl aluminum dichloride as a Lewis acid. The reduction of the ester (**3**) to the allylic alcohol (**4**) proceeds with diisobutyl aluminum hydride in tetrahydrofuran solution. The formation of the epoxide (**5**) from the allylic alcohol (**4**) results from a Sharpless epoxidation using D-(-)-diethyl tartrate, titanium(IV)- isopropoxide and tert-butyl hydroperoxide in the presence of activated 4A sieves in dichloromethane solution.
The reduction of the epoxide (**5**) to the 1,3-diol (**6**) can be effected using Red-Al™ (Aldrich) as reducing agent. The diol (**6**) can be selectively converted to the mono-benzoate (**7**) with benzoyl chloride in the presence of pyridine in dichloromethane solution. The replacement of the hydroxy group of (**7**) with fluorine to give the fluoro-ester (**8**) with an inversion of configuration is expected from the reaction of (**7**) with diethylaminosulfur trifluoride (DAST) at -92° C. The fluoro-ester (**8**) can be hydrolyzed to the fluoro-alcohol (9) with lithium methoxide in methanol. The oxidation of the fluoro-alcohol (**9**) to the fluoro-aldehyde (**10**) can be achieved by reaction with oxalyl chloride, dimethyl sulfoxide and triethylamine at -70° C to room temperature.
The cyclization of the fluoro-aldehyde (**10**) to the fluorocyclohexanol (**11**), an ene reaction, can be promoted with a reagent obtained from 2,6-di-tert-butyl-4-methyl-phenol and trimethyl aluminum as a Lewis acid at -78° in dichloromethane solution. The newly formed hydroxy group of (**11**) is protected as tert-butyl dimethylsilyl (**12**) by a reaction of (**11**) with imidazole and tert-butyl-dimethylsilyl chloride in dimethylformamide as a solvent at room temperature. The removal of the benzyl protecting group of (**12**) to give the allylic alcohol (**13**) is achievable by reduction with lithium di-tert-butylbiphenylide in tetrahydrofuran solution at -78°. The conversion of the allylic alcohol (**13**) to the diphenyl phosphate oxide (**15**) can be done using known methods.

As described above, also provided are intermediates for synthesizing compounds of Formula (**I)**. These intermediates are compounds of Formula (**II**) 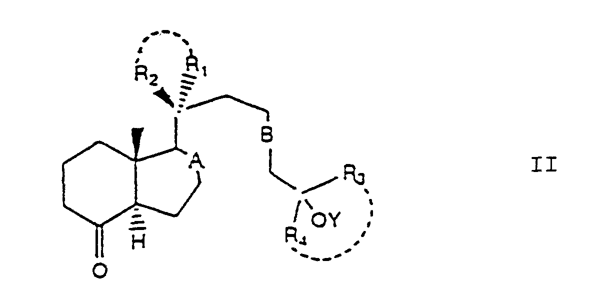 wherein:
R₁ and R₂ are
   (a) both methyl,
   (b) together with the carbon to which they are attached form a cyclopropane ring, or
   (c) form a =CH₂;
R₃ and R₄ are, independently of each other, a (C₁-C₄) alkyl or fluoroalkyl, or R₃ and R₄ together with C-25 form a (C₃-C₉) cycloalkyl or cyclofluoroalkyl;
A is a single bond or a double bond;
B is a double bond or a triple bond; and
Y is H or OSiMe₃.

The compounds of this invention are useful for the prevention and treatment of a variety of mammalian conditions manifested by loss of bone mass. In particular, the compounds of this invention are indicated for the prophylaxis and therapeutic treatment of osteoporosis and osteopenia in mammals without inducing hypercalciuria, hypercalcemia, or nephrotoxicity. As used herein, "hypercalciuria" is excessive calcium in the urine, in humans corresponding to an excretion of greater than 4 mg/kg/day: This often results in nephrolithiasis (renal calculi). "Hypercalcemia" is an excessive concentration of calcium in the serum; in humans (and rats) this corresponds to greater than about 10.5 mg/dl. "Intolerable hypercalcemia", usually occurring at serum calcium concentrations greater than about 12 mg/dl, is associated with emotional lability, confusion, delirium, psychosis, stupor, and coma.
The compounds of this invention are expected to be useful in the treatment of Type I (postmenopausal), Type II (senile), and Type III (iatrogenic) osteoporosis, including that associated with immunosuppressive drugs used in organ transplantation, as well in the treatment of osteodystrophy due to renal dialysis and hyperparathyroidism.

In general, the compound of this invention may be administered in amounts between about 0.0002 and 0.5 µg compound/kg body weight per day, preferably from about 0.001 to about 0.1 µg/kg body weight per day, most preferably from about 0.002 to about 0.02 µg/kg body weight per day. For a 50 kg human subject, the daily dose of active ingredient may be from about 0.01 to about 25 µgs, preferably from about 0.05 to about 10 µgs, most preferably from about 0.1 µg to about 1µg per day. In other mammals, such as horses, dogs, and cattle, other doses may be required. This dosage may be delivered in a conventional pharmaceutical composition by a single administration, by multiple applications, or via controlled release, as needed to achieve the most effective results, preferably once or twice daily by mouth. In certain situations, alternate day dosing may prove adequate to achieve the desired therapeutic response.
The selection of the exact dose and composition and the most appropriate delivery regimen will be influenced by, *inter alia,* the pharmacological properties of the formulation, the nature and severity of the condition being treated, and the physical condition and mental acuity of the recipient. In the treatment of corticosteroid induced osteopenia, it is expected that the requisite dose will be greater for higher doses of corticosteroids.
Representative delivery regimens include oral, parenteral (including subcutaneous, intramuscular and intravenous), rectal, buccal (including sublingual), pulmonary, transdermal, and intranasal, most preferably oral.
A further aspect of the present invention relates to pharmaceutical compositions comprising as an active ingredient a compound of the present invention, in admixture with a pharmaceutically acceptable, non-toxic carrier. As mentioned above, such compositions may be prepared for parenteral (subcutaneous, intramuscular or intravenous) administration, particularly in the form of liquid solutions or suspensions; for oral or buccal administration, particularly in the form of tablets or capsules; for pulmonary or intranasal administration, particularly in the form of powders, nasal drops or aerosols; and for rectal or transdermal administration.
The compositions may conveniently be administered in unit dosage form and may be prepared by any of the methods well-known in the pharmaceutical art, for example as described in *Remington's Pharmaceutical Sciences,* 17th ed., Mack Publishing Company, Easton, PA., (1985). Formulations for parenteral administration may contain as excipients sterile water or saline, alkylene glycols such as propylene glycol, polyalkylene glycols such as polyethylene glycol, oils of vegetable origin, hydrogenated naphthalenes and the like. Formulations for nasal administration may be solid and may contain excipients, for example, lactose or dextran, or may be aqueous or oily solutions for use in the form of nasal drops or metered spray. For buccal administration typical excipients include sugars, calcium stearate, magnesium stearate, pregelatinated starch, and the like.
Orally administrable compositions may comprise one or more physiologically compatible carriers and/or excipients and may be in solid or liquid form, including, for example, tablets, coated tablets, capsules, lozenges, aqueous or oily suspensions, solutions, emulsions, elixirs, and powders suitable for reconstitution with water or another suitable liquid vehicle before use. Tablets and capsules may be prepared with binding agents, for example, syrup, acacia, gelatin, sorbitol, tragacanth, or poly-vinylpyrollidone; fillers, such as lactose, sucrose, corn starch, calcium phosphate, sorbitol, or glycine; lubricants, such as magnesium stearate, talc, polyethylene glycol, or silica; and surfactants, such as sodium lauryl sulfate. Liquid compositions may contain conventional additives such as suspending agents, for example sorbitol syrup, methyl cellulose, sugar syrup, gelatin, carboxymethylcellulose, or edible fats; emulsifying agents such as lecithin, or acacia; vegetable oils such as almond oil, coconut oil, cod liver oil, or peanut oil; preservatives such as butylated hydroxyanisole (BHA) and butylated hydroxytoluene (BHT). Liquid compositions may be encapsulated in, for example, gelatin to provide a unit dosage form.
Preferred solid oral dosage forms include tablets, two- piece hard shell capsules and soft elastic gelatin (SEG) capsules. SEG capsules are of particular interest because they provide distinct advantages over the other two forms (see Seager, H., "Soft gelatin capsules: a solution to many tableting problems"; *Pharmaceutical Technology,* 9, (1985). Some of the advantages of using SEG capsules are: a) dosecontent uniformity is optimized in SEG capsules because the drug is dissolved or dispersed in a liquid that can be dosed into the capsules accurately b) drugs formulated as SEG capsules show good bioavailability because the drug is dissolved, solubilized or dispersed in an aqueous-miscible or oily liquid and therefore when released in the body the solutions dissolve or are emulsified to produce drug dispersions of high surface area and c) degradation of drugs that are sensitive to oxidation during long-term storage is prevented because the dry shell of soft gelatin provides a barrier against the diffusion of oxygen.
The dry shell formulation typically comprises of about 40% to 60% concentration of gelatin, about a 20% to 30% concentration of plasticizer (such as glycerin, sorbitol or propylene glycol) and about a 30 to 40% concentration of water. Other materials such as preservatives, dyes, opacifiers and flavours also may be present. The liquid fill material comprises a solid drug that has been dissolved, solubilized or dispersed (with suspending agents such as beeswax, hydrogenated castor oil or polyethylene glycol 4000) or a liquid drug in vehicles or combinations of vehicles such as mineral oil, vegetable oils, triglycerides, glycols, polyols and surface-active agents.
Also provided are methods for the manufacture of a medicament for treating osteoporosis and other bone related diseases, particularly those related to the loss of bone mass, using a compound of Formula (**I**), wherein: X is =CH₂; one of R₁ and R₂ is hydrogen, the other is (C₁-C₄) alkyl; R₃ and R₄ are (C₁-C₄) alkyl; A is a double bond; and B is a double bond.
Pharmaceutical compositions, dosages and delivery regimens for these methods of treatment are as described earlier. The methods are of particular utility in treating osteoporosis in a human female.
Preferred embodiments of the method are use of a compound of Formula I, wherein:
X is =CH₂;
   one of R₁ and R₂ is hydrogen and the other is CH₃;
   R₃ and R₄ are each C₂H₅;
A is a double bond; and
B is a trans double bond.
Pharmaceutical compositions, dosages and delivery regimens for these methods of treatment are as described earlier. The methods are of particular utility in treating osteoporosis in a human female.

### EXAMPLES

The following examples are given to enable those skilled in the art to more clearly understand and to practice the present invention. They should not be considered as limiting the scope of the invention, but merely as being illustrative and representative thereof.
It should be noted that although the following synthetic examples specifically describe the preparations of a compound of Formula (**I**) from intermediate II where Y is hydrogen, that compound (**I**) can also be prepared from the corresponding silylated derivative of intermediate II (Y = SiMe₃) under the same reaction conditions.

### Example 1 (Comparative)

### 1α-Fluoro-25-hydroxy-23-yne-26,27-hexafluoro-20-methylcholecalciferol

The title compound can be prepared by the following procedure:

To a solution of 1.5 mmole (3S-(3α,5β,Z)]-2-[2-[2-methylene-3-fluoro-5-[[(1,1-dimethylethyl)dimethylsilyl]oxy] cyclohexylidene]ethyl]diphenyl phosphine oxide (III) in 9 ml anhydrous tetrahydrofuran at -78°C is added 1.5 mmole of 1.6 M n-butyllithium in hexane dropwise in an argon atmosphere. After stirring for 5 minutes, a solution of 0.6 mmole 3aR-(3aα,7aβ)-1,2,3,3a,5,6,7,7a-octahydro-7a-methyl-1-[6,6,6-trifluoro-5-hydroxy-5-trifluoromethyl-1,1-dimethyl-3-hexynyl]-4H-inden-4-one (IIa) in 4 ml of anhydrous tetrahydrofuran is added dropwise over a 10 min period. The reaction mixture is then stirred at -78°C for 2 hrs., quenched with 2N Rochelle salt and warmed up to room temperature. The isolation of the silylated title compound can be done by extraction with ethyl acetate and purification by FLASH chromatography.
Removal of the silyl protecting group can be performed by treating a solution of the silylated title compound in 4 ml of anhydrous tetrahydrofuran with 1.25 mmole of 1M solution of tetrabutylammonium fluoride in tetrahydrofuran under argon and stirring at room temperature for 18 hrs. This reaction mixture is quenched with water and the resulting title compound is isolated by extraction with ethyl acetate and purification by FLASH chromatography.

### Example 2

### 1α-Fluoro-25-hydroxy-23-yne-26,27-hexafluoro-20,21,28-cyclopropyl-cholecalciferol

The title compound can be prepared by the following procedure:

To a solution of 1.5 mmole (3S-(3α,5β,Z)]-2-[2-[2-methylene-3-fluoro-5-[[(1,1-dimethylethyl)dimethylsilyl]oxy] cyclohexylidene]ethyl]diphenyl phosphine oxide (III) in 9 ml anhydrous tetrahydrofuran at -78°C is added 1.5 mmole of 1.6 M n-butyllithium in hexane dropwise in an argon atmosphere. After stirring for 5 minutes, a solution of 0.6 mmole 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5-octynyl]-4H-inden-4-one (IIb) in 4 ml of anhydrous tetrahydrofuran is added dropwise over a 10 min period. The reaction mixture is then stirred at -78°C for 2 hrs., quenched with 2N Rochelle salt and warmed up to room temperature. The isolation of the silylated title compound can be done by extraction with ethyl acetate and purification by FLASH chromatography.
Removal of the silyl protecting group can be performed by treating a solution of the silylated title compound in 4 ml of anhydrous tetrahydrofuran with 1.25 mmole of 1M solution of tetrabutylammonium fluoride in tetrahydrofuran under argon and stirring at room temperature for 18 hrs. This reaction mixture is quenched with water and the resulting title compound is isolated by extraction with ethyl acetate and purification by FLASH chromatography.

### Example 3 (Comparative)

### 1α-Fluoro-25-hydroxy-20-ene-23-yne-26,27-hexafluorocholecalciferol

The title compound can be prepared by the following procedure:

To a solution of 1.5 mmole (3S-(3α,5β,Z)]-2-[2-[2-methylene-3-fluoro-5-[[(1,1-dimethylethyl)dimethylsilyl]oxy] cyclohexylidene]ethyl]diphenyl phosphine oxide (III) in 9 ml anhydrous tetrahydrofuran at -78°C is added 1.5 mmole of 1.6 M n-butyllithium in hexane dropwise in an argon atmosphere. After stirring for 5 minutes, a solution of 0.6 mmole 3aR-(3aα,7aβ)-1,2,3,3a,5,6,7,7a-octahydro-7a-methyl-1-[6,6,6-trifluoro-5-hydroxy-5-trifluoromethyl-1-methylene-3-hexynyl]-4H-inden-4-one (IIc) in 4 ml of anhydrous tetrahydrofuran is added dropwise over a 10 min period. The reaction mixture is then stirred at -78°C for 2 hrs., quenched with 2N Rochelle salt and warmed up to room temperature. The isolation of the silylated title compound can be done by extraction with ethyl acetate and purification by FLASH chromatography.
Removal of the silyl protecting group can be performed by treating a solution of the silylated title compound in 4 ml of anhydrous tetrahydrofuran with 1.25 mmole of 1M solution of tetrabutylammonium fluoride in tetrahydrofuran under argon and stirring at room temperature for 18 hrs. The reaction mixture is quenched with water and the resulting title compound is isolated by extraction with ethyl acetate and purification by FLASH chromatography.

### Example 4 (Comparative)

### 1α-Fluoro-25-hydroxy-23-yne-20-methyl-26,27-bishomo-26,26a,27,27a-decafluoro-cholecalciferol

The title compound can be prepared by the following procedure:

To a solution of 1.5 mmole (3S-(3α,5β,Z)]-2-[2-[2-methylene-3-fluoro-5-[[(1,1-dimethylethyl)dimethylsilyl]oxy]-cyclohexylidene]ethyl]diphenyl phosphine oxide (III) in 9 ml anhydrous tetrahydrofuran at -78°C is added 1.5 mmole of 1.6 M n-butyllithium in hexane dropwise in an argon atmosphere. After stirring for 5 minutes, a solution of 0.6 mmole 3aR-(3aα,7aβ)-1,2,3,3a,5,6,7,7a-octahydro-7a-methyl-1-[6,6,7,7,7-pentafluoro-5-hydroxy-5-pentafluoroethyl-1,1-dimethyl-3-heptynyl]-4H-inden-4-one (IId) in 4 ml of anhydrous tetrahydrofuran is added dropwise over a 10 min period. The reaction mixture is then stirred at -78°C for 2 hrs., quenched with 2N Rochelle salt and warmed up to room temperature. The isolation of the silylated title compound can be done by extraction with ethyl acetate and purification by FLASH chromatography.
Removal of the silyl protecting group can be performed by treating a solution of the silylated title compound in 4 ml of anhydrous tetrahydrofuran with 1.25 mmole of 1M solution of tetrabutylammonium fluoride in tetrahydrofuran under argon and stirring at room temperature for 18 hrs. The reaction mixture is quenched with water and the resulting title compound is isolated by extraction with ethyl acetate and purification by FLASH chromatography.

### Example 5 (Comparative)

### 1α-Fluoro-25-hydroxy-16-ene-23-yne-26,27-hexafluoro-20-methylcholecalciferol

The title compound can be prepared by following the same experimental design as in the Example 1 and using instead of 3aR-(3aα,7aβ)-1,2,3,3a,5,6,7,7a-octahydro-7a-methyl-[6,6,6-trifluoro-5-hydroxy-5-trifluoromethyl-1,1-dimethyl-3-hexynyl]-4H-inden-4-one (IIa) the corresponding 1-ene derivative, 3aR-(3aα,7aβ)-3,3a,5,6,7,7a-hexahydro-7a-methyl-[6,6,6-trifluoro-5-hydroxy-5-trifluoromethyl-1,1-dimethyl-3-hexy-nyl]-4H-inden-4-one (IIe).

### Example 6

### 1α-Fluoro-25-hydroxy-16-ene-23-yne-26,27-hexafluoro-20,21,28-cyclopropyl-cholecalciferol

The title compound can be prepared by following the same experimental design as in the Example 2 and using instead of 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5-octynyl]-4H-inden-4-one (IIb) the corresponding 1-ene derivative, 3aR-(3aα,7aβ)-3,3a,5,6,7,7a-hexahydro-7a-methyl-1-[8,8,8-trifluoro-7-hydroxy-7-trifluoromethyl-1,3-cyclo-5-octynyl]-4H-inden-4-one (IIf).

### Example 7 (Comparative)

### 1α-Fluoro-25-hydroxy-16-ene-23-yne-20-methyl-26,27-bishomo-26a,27a-hexafluoro-cholecalciferol

The title compound can be prepared by following the same experimental design as in the Example 4 and using instead of 3aR-(3aα,7aβ)-1,2,3,3a,5,6,7,7a-octahydro-7a-methyl-1-[6,6,7,7,7-pentafluoro-5-hydroxy-5-pentafluoroethyl-1,1-dimethyl-3-heptynyl]-4H-inden-4-one (IId) the corresponding 1-ene-hexafluoro derivative, 3aR-(3aα,7aβ)-3,3a,5,6,7,7a-hexahydro-7a-methyl-1-[7,7,7-trifluoro-5-hydroxy-5-trifluoroethyl-1,1-dimethyl-3-heptynyl]-4H-inden-4-one (IIg).

### Example 8

### 1α-Fluoro-25-hydroxy-23Z-ene-26,27-hexafluoro-20-methyl-cholecalciferol (Comparative)

The title compound can be prepared by following the same experimental design as in the Example 1 and using instead of 3aR-(3aα,7aβ)-1,2,3,3a,5,6,7,7a-octahydro-7a-methyl-1-[6,6,6-trifluoro-5-hydroxy-5-trifluoromethyl-1,1-dimethyl-3-hexynyl]-4H-inden-4-one (IIa) the corresponding 3Z-hexenyl derivative, 3aR-(3aα,7aβ)-1,2,3,3a,5,6,7,7a-octa-hydro-7a-methyl-1-[6,6,6-trifluoro-5-hydroxy-5-trifluoromethyl-1,1-dimethyl-3Z-hexenyl]-4H-inden-4-one (IIh).

### Example 9

### 1α-Fluoro-25-hydroxy-23Z-ene-26,27-hexafluoro-20,21,28-cyclopropyl-cholecalciferol

The title compound can be prepared by following the same experimental design as in the Example 2 and using instead of 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5-octynyl]-4H-inden-4-one (IIb) the corresponding 5Z-octenyl derivative, 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5Z-octenyl]-4H- inden-4-one (IIi).

### Example 10 (Comparative)

### 1α-Fluoro-25-hydroxy-20,23Z-diene-26,27-hexafluorocholecalciferol

The title compound can be prepared by following the same experimental design as in the Example 3 and using instead of 3aR-(3aα,7aβ)-1,2,3,3a,5,6,7,7a-octahydro-7a-methyl-1-[6,6,6-trifluoro-5-hydroxy-5-trifluoromethyl-1-methylene-3-hexynyl]-4H- inden-4-one (IIc) the corresponding 3Z-hexenyl derivative, 3aR-(3aα,7aβ)-1,2,3,3a,5,6,7,7a-octahydro-7a-methyl-1-[6,6,6-trifluoro-5-hydroxy-5-tri-fluoro-methyl-1-methylene-3Z-hexenyl]- 4H-inden-4-one (IIj).

### Example 11 (Comparative)

### 1α-Fluoro-25-hydroxy-23Z-ene-20-methyl-26,27-bishomo-26,26a,27,27a-decafluoro-cholecalciferol

The title compound can be prepared by following the same experimental design as in the Example 4 and using instead of 3aR-(3aα,7aβ)-1,2,3,3a,5,6,7,7a-octahydro-7a-methyl-1-[6,6,7,7,7-pentafluoro-5-hydroxy-5-pentafluoroethyl-1,1-dimethyl-3-heptynyl]-4H-inden-4-one (IId) the corresponding 3Z-heptynyl derivative, 3aR-(3aα,7aβ)-1,2,3,3a,5,6,7,7a-octahydro-7a-methyl-1-[6,6,7,7,7-pentafluoro-5-hydroxy-5-pentafluoroethyl- 1,1-dimethyl-3Z-heptenyl]-4H-inden-4-one (IIk).

### Example 12 (Comparative)

### 1α-Fluoro-25-hydroxy-16,23Z-diene-26,27-hexafluoro-20-methyl-cholecalciferol

The title compound can be prepared by following the same experimental design as in the Example 1 and using instead of 3aR-(3aα,7aβ)-1,2,3,3a,5,6,7,7a-octahydro-7a-methyl-1-[6,6,6-trifluoro-5-hydroxy-5-trifluoromethyl-1,1-dimethyl-3-hexynyl]-4H-inden-4-one (IIa) the corresponding 1,32-diene derivative, 3aR-(3aα,7aβ)-3,3a,5,6,7,7a-hexahydro-7a-methyl-1-[6,6,6-trifluoro-5-hydroxy-5-trifluoromethyl-1,1-dimethyl-3Z-hexenyl]-4H-inden-4-one (IIl).

### Example 13

### 1α-Fluoro-25-hydroxy-16,23Z-diene-26,27-hexafluoro-20,21,28-cyclopropyl-cholecalciferol

The title compound can be prepared by following the same experimental design as in Example 2 and using instead of 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5-octynyl]-4H-inden-4-one (IIb) the corresponding 1,52-diene derivative, 3aR-(3aα,7aβ)-3,3a,5,6,7,7a-hexahydro-7a-methyl-1-[8,8,8-trifluoro-7-hydroxy-7-trifluoromethyl-1,3-cyclo-5Z-octenyl]-4H-inden-4-one (IIm).

### Example 14 (Comparative)

### 1α-Fluoro-25-hydroxy-16,23Z-diene-20-methyl-26,27-bishomo-26a,27a-hexafluoro-cholecalciferol

The title compound can be prepared by following the same experimental design as in the Example 4 and using instead of 3aR-(3aα,7aβ)-1,2,3,3a,5,6,7,7a-octahydro-7a-methyl-1-[6,6,7,7,7-pentafluoro-5-hydroxy-5-pentafluoroethyl-1,1-dimethyl-3-heptynyl]-4H-inden-4-one (IId) the corresponding 1,3Z-diene-hexafluoro derivative, 3aR-(3aα,7aβ)-3,3a,5,6,7,7a-hexahydro-7a-methyl-1-[7,7,7-trifluoro-5-hydroxy-5-trifluoroethyl-1,1-dimethyl-3Z-heptenyl]-4H-inden-4-one (IIn).

### Example 15 (Comparative)

### 1α-Fluoro-25-hydroxy-23E-ene-26,27-hexafluoro-20-methyl-cholecalciferol

The title compound can be prepared by following the same experimental design as in the Example 1 and using instead of 3aR-(3aα,7aβ)-1,2,3,3a,5,6,7,7a-octahydro-7a-methyl-1-[6,6,6-trifluoro-5-hydroxy-5-trifluoromethyl-1,1-dimethyl-3-hexynyl]-4H-inden-4-one (IIa) the corresponding 3E-hexenyl derivative, 3aR-(3aα,7aβ)-1,2,3,3a,5,6,7,7a-octahydro-7a-methyl-1-[6,6,6-trifluoro-5-hydroxy-5-tri-fluoromethyl-1,1-dimethyl-3E-hexenyl]-4H-inden-4-one (IIo).

### Example 16

### 1α-Fluoro-25-hydroxy-23E-ene-26,27-hexafluoro-20,21,28-cyclopropyl-cholecalciferol

The title compound can be prepared by following the same experimental design as in the Example 2, and using instead of 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5-octynyl]-4H-inden-4-one (IIb) the corresponding 5E-octenyl derivative, 3aR-(3aα,7aβ)-1,2,3,3a,5,6,7,7a-octahydro-7a-methyl-1-[8,8,8-trifluoro-7-hydroxy-7-tri-fluoromethyl-1,3-cyclo-5E-octenyl]-4H-inden-4-one (IIp).

### Example 17 (Comparative)

### 1α-Fluoro-25-hydroxy-20,23E-diene-26,27-hexafluorocholecalciferol

The title compound can be prepared by following the same experimental design as in the Example 3, and using instead of 3aR-(3aα,7aβ)-1,2,3,3a,5,6,7,7a-octahydro-7a-methyl-1-[6,6,6-trifluoro-5-hydroxy-5-trifluoromethyl-1-methylene-3-hexynyl]-4H- inden-4-one (IIc) the corresponding 3E-hexenyl derivative, 3aR-(3aα,7aβ)-1,2,3,3a,5,6,7,7a-octahydro-7a-methyl-1-[6,6,6-trifluoro-5-hydroxy-5-tri-fluoromethyl-1-methylene-3-hexenyl]-4H-inden-4-one (IIq).

### Example 18 (Comparative)

### 1α-Fluoro-25-hydroxy-23E-ene-20-methyl-26,27-bishomo-26,26a,27,27a-decafluoro-cholecalciferol

The title compound can be prepared by following the same experimental design as in the Example 4, and using instead of 3aR-(3aα,7aβ)-1,2,3,3a,5,6,7,7a-octahydro-7a-methyl-1-[6,6,7,7,7-pentafluoro-5-hydroxy-5-pentafluoroethyl-1,1-dimethyl-3-heptynyl]-4H-inden-4-one (IId) the corresponding 3E-heptenyl derivative, 3aR-(3aα,7aβ)-1,2,3,3a,5,6,7,7a-octahy-dro-7a-methyl-1-[6,6,7,7,7-pentafluoro-5-hydroxy-5-pentafluoro-1,1-di-methyl-3E-heptenyl]-4H-inden-4-one (IIr).

### Example 19 (Comparative)

### 1α-Fluoro-25-hydroxy-16,23E-diene-26,27-hexafluoro-20-methyl-cholecalciferol

The title compound can be prepared by following the same experimental design as in the Example 1, and using instead of 3aR-(3aα,7aβ)-1,2,3,3a,5,6,7,7a-octahydro-7a-methyl-1-[6,6,6-trifluoro-5-hydroxy-5-trifluoroethyl-1,1-dimethyl-3-hexynyl]-4H-inden-4-one (IIa) the corresponding 1,3E-diene derivative, 3aR-(3aα,7aβ)-3,3a,5,6,7,7a-hexahydro-7a-methyl-1-[6,6,6-trifluoro-5-hydroxy-5-trifluoromethyl-1,1-dimethyl-3E-hexenyl]-4H-inden-4-one (IIs).

### Example 20

### 1α-Fluoro-25-hydroxy-16,23E-diene-26,27-hexafluoro-20,21,28-cyclopropyl-cholecalciferol

The title compound can be prepared by following the same experimental design as in the Example 2, and using instead of 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5-octynyl]-4H-inden-4-one (IIb) the corresponding 1,5E-diene derivative, 3aR-(3aα,7aβ)-3,3a,5,6,7,7a-hexahydro-7a-methyl-1-[8,8,8-trifluoro-7-hydroxy-7-trifluoromethyl-1,3-cyclo-5E-octenyl]-4H-inden-4-one (IIt).

### Example 21 (Comparative)

### 1α-Fluoro-25-hydroxy-16,23E-diene-20-methyl-26,27-bishomo-26a,27a-hexafluoro-cholecalciferol

The title compound can be prepared by following the same experimental design as in the Example 4, and using instead of 3aR-(3aα,7aβ)-1,2,3,3a,5,6,7,7a-octahydro-7a-methyl-1-[6,6,7,7,7-pentafluoro-5-hydroxy-5-pentafluoroethyl-3-heptynyl]-4H-inden-4-one (IId), the corresponding 1,3E-diene-hexafluoro derivative, 3aR-(3aα,7aβ)-3,3a,5,6,7,7a-hexahydro-7a-methyl-1-[7,7,7-trifluoro-5-hydroxy-5-trifluoroethyl-3E-heptenyl]-4H-inden-4-one (IIu).

### Example 22 (Comparative)

### 1α-Fluoro-25-hydroxy-16,20-diene-23-yne-26,27-hexafluorocholecalciferol

The title compound can be prepared by following the same experimental design as in the Example 3, and using instead of 3aR-(3aα,7aβ)-1,2,3,3a,5,6,7,7a-octahydro-7a-methyl-1-[6,6,6-trifluoro-5-hydroxy-5-trifluoromethyl-1-methylene-3-hexenyl]-4H-inden-4-one (IIc), the corresponding 1-ene derivative, 3aR-(3aα,7aβ)-3,3a,5,6,7,7a-hexahydro-7a-methyl-1-[6,6,6-trifluoro-5-hydroxy-5-trifluoromethyl-1-methylene-hexynyl]-4H-inden-4-one (IIv).

### Example 23 (Comparative)

### 1α-Fluoro-25-hydroxy-16,20,23Z-triene-26,27-hexafluorocholecalciferol

The title compound can be prepared by following the same experimental design as in the Example 3, and using instead of 3aR-(3aα,7aβ)-1,2,3,3a,5,6,7,7a-octahydro-7a-methyl-1-[6,6,6-trifluoro-5-hydroxy-5-trifluoromethyl-1-methylene-3-hexenyl]-4H-inden-4-one (IIc) the corresponding 1,32-diene derivative, 3aR-(3aα,7aβ)-3,3a,5,6,7,7a-hexahydro-7a-methyl-1-[6,6,6-trifluoro-5-hydroxy-5-trifluoromethyl-1-methylene-3Z-hexenyl]-4H-inden-4-one (IIw).

### Example 24 (Comparative)

### 1α-Fluoro-25-hydroxy-16,20,23E-triene-26,27-hexafluorocholecalciferol

The title compound can be prepared by following the same experimental design as in the Example 3, and using instead of 3aR-(3aα,7aβ)-1,2,3,3a,5,6,7,7a-octahydro-7a-methyl-1-[6,6,6-trifluoro-5-hydroxy-5-trifluoromethyl-1-methylene-3-hexynyl]-4H-inden-4-one (IIc), the corresponding 1,3E-diene derivative, 3aR-(3aα,7aβ)-3,3a,5,6,7,7a-hexahydro-7a-methyl-[6,6,6-trifluoro-5-hydroxy-5-trifluoromethyl-1-methylene-3E-hexenyl]-4H-inden-4-one (IIz).

### Example 25

### 1α-Fluoro-25-hydroxy-23E-ene-26,27-hexafluoro-20,21,28-cyclopropyl-19-nor-cholecalciferol

The title compound can be prepared by the following procedure:

To a solution of 1.5 mmole (3R-(3α,5β,Z)]-2-[2-[3-fluoro-5-[[(1,1-dimethylethyl)dimethylsilyl]oxy]cyclohexylidene]-ethyl]diphenyl phosphine oxide (**15**) in 9 ml anhydrous tetrahydrofuran at -78° C is added 1.5 mmole of 1.6 M n-butyllithium in hexane dropwise in an argon atmosphere. After stirring for 5 minutes, a solution of 0.6 mmole 3aR-(3aα,7aβ)-1,2,3,3a,5,6,7,7a-octahydro-7a-methyl-1-[8,8,8-trifluoro-7-hydroxy-7-trifluoromethyl-1,3-cyclo-5E-octenyl]-4H-inden-4-one (IIp) in 4 ml of anhydrous tetrahydrofuran is added dropwise over a 10 min period. The reaction mixture is then stirred at -78° C for 2 hours, quenched with 2N Rochelle salt and warmed up to a room temperature. The isolation of the silylated title compound can be done by extraction with ethyl acetate and purification by FLASH chromatography.

Removal of the silyl protecting group can be performed by treating a solution of the silylated title compound in 4 ml of anhydrous tetrahydrofuran with 1.25 mmole of 1M solution of tetrabutylammonium fluoride in tetrahydrofuran under argon and stirring at room temperature for 18 hrs. This reaction mixture is quenched with water and the resulting title compound is isolated by extraction with ethyl acetate and purification by FLASH chromatography.

It will be recognized by those skilled in the art that there are different variations of these syntheses or alternative synthetic methods which are well known in the art that may be employed to achieve some steps in the syntheses detailed above.

### Example 26

### Bone anabolism in the rat

The compounds of the present invention are more effective than 1,25-dihydroxy vitamin D₃ at bone accretion and do not induce hypercalciuria, nephrotoxicity, or hypercalcemia at therapeutically effective doses. This has been demonstrated as follows:

Three month old rats are ovariectomized (Ovx) and administered either 1,25-dihydroxy vitamin D₃ (vit. D in Table) or one of the compounds of the present invention once a day by mouth starting at 3 weeks post-ovariectomy and continuing until final sacrifice at 6 weeks post-ovariectomy. Control groups, both sham (rats that were not ovariectomized) and Ovx, received vehicle only. Blood and urine samples were collected twice, at 4 weeks post-ovariectomy and again at the 6 week mark and the amount of serum and urine calcium was determined. The final femoral calcium determined upon sacrifice 6 weeks post- ovariectomy.

The bone mineral density (BMD) of the right femur was determined by using a High Resolution Software Package on a QDR-1000W Bone Densitometer™ (Hologic, Walthan, MA). The animals were scanned by placing them on a scanning block in a supine position such that the right leg was perpendicular to the main body and the tibia was perpendicular to the femur.

The increase in the bone mineral density and the amount of calcium in the urine and the serum for some of the compounds of this invention in this assay are given in the table below:

| CPD # | Surgery Treatment Dose µg/kg | | | Whole Femur BMD mg/cm² | Serum Calcium g/dl (6th wk) | Urine Calcium/Creatinine mg/dl 6th wk) |
|---|---|---|---|---|---|---|
| 57* | Sham | Vehicle | 0.00 | 0.249 | 10.41 | 0.40 |
| | Ovx | Vehicle | 0.00 | 0.23 | 9.81 | 0.26 |
| | Ovx | Vit D | 0.20 | 0.2370 | 10.21 | 1.35 |
| | Ovx | Cpd. 57 | 1.50 | 0.248a | 10.12 | 0.90 |
| 58 | Sham | Vehicle | 0.00 | 0.251 | 9.27 | 0.35 |
| | Ovx | Vehicle | 0.00 | 0.228 | 9.70 | 0.29 |
| | Ovx | Vit D | 0.20 | 0.233 | 10.93a | 1.44 |
| | Ovx | Cpd. 58 | 5.00 | 0.236a | 9.46 | 0.63 |
| 59* | Sham | Vehicle | 0.00 | 0.244 | 8.74 | 0.38 |
| | Ovx | Vehicle | 0.00 | 0.233 | 8.81 | 0.30 |
| | Ovx | Vit. D | 0.20 | 0.235 | 9.51a | 0.76 |
| | Ovx | Cpd. 59 | 2.00 | 0.233 | 9.20 | 0.35 |
| 60 | Sham | Vehicle | 0.00 | 0.255 | 9.09 | 0.23 |
| | Ovx | Vehicle | 0.00 | 0.234 | 9.54 | 0.17 |
| | Ovx | Vit. D | 0.20 | 0.240 | 10.64a | 1.44 |
| | Ovx | Cpd. 60 | 0.10 | 0.242 | 9.59 | 0.52 |
| 61 | Sham | Vehicle | 0.00 | 0.239 | 9.20 | 0.31 |
| | Ovx | Vehicle | 0.00 | 0.224 | 9.31 | 0.27 |
| | Ovx | Vit. D | 0.20 | 0.239a | 10.61a | 1.48 |
| | Ovx | Cpd. 61 | 0.50 | 0.243a | 10.68a | 1.34 |

| | | | | | | |
|---|---|---|---|---|---|---|
| a = Significant difference (p < 0.05), Cpd. or Vit. D vs Ovx vehicle. | | | | | | |
| * = Not claimed | | | | | | |

As seen above the compounds of this invention, in general, increase the bone mineral density but show reduced calciuria and calcemia than 1,25-di(OH) vitamin D.

### Example 27

### Oral dosage form soft gelatin capsule

A capsule for oral administration is formulated under nitrogen in amber light from 0.01 to 25.0 µg of one of the compounds of the present invention in 150 mg of fractionated coconut oil, with 0.015 mg butylated hydroxytoluene (BHT) and 0.015 mg butylated hydroxyanisole (BHA), filled in a soft gelatin capsule.

## Claims

1. The compound the formula 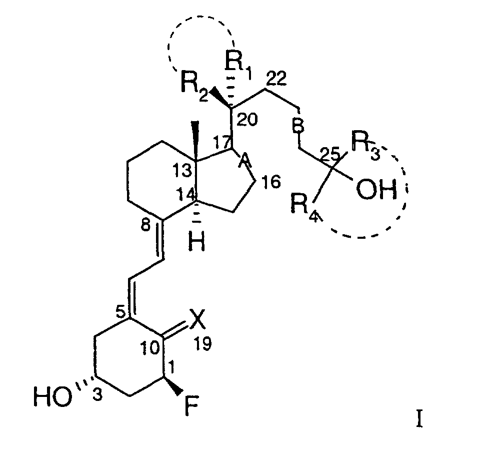 wherein:
X is (H,H) or =CH₂;
R₁ and R₂ together with C-20 form a cyclopropano, cyclodifluoropropano or cyclotetrafluoropropano group;
R₃ and R₄ are, independently of each other, selected from the group consisting of CH₃, CF₃, CH₂CH₃, CH₂CF₃, CF₂CH₃ and CF₂CF₃;
A is a single bond or a double bond; and
B is a double bond or a triple bond.

2. The compound of Claim 1, wherein:
X is =CH₂;
R₁ and R₂ together with C-20 form a cyclopropano, cyclodifluoropropano or cyclotetrafluoropropano group;
R₃ and R₄ are, independently of each other, selected from the group consisting of CH₃, CF₃, CH₂CH₃, CH₂CF₃, CF₂CH₃ and CF₂CF₃;
A is a single bond or a double bond; and
B is a double bond or a triple bond.

3. The compound of Claim 2, wherein:
R₁ and R₂ together with C-20 form a cyclopropano group; R₃ and R₄ are each CF₃;
A is a double bond; and
B is a triple bond.

4. A pharmaceutical composition comprising a pharmaceutically acceptable carrier and a compound of formula (I) as in claim 1.

5. A process for the manufacture of a compounds of formula I as in claim 1, which comprises removing the silyl protecting group(s) contained from a silylated compound of formula I.

6. Compounds of formula I as in claim 1 for use as therapeutically active agents, particularly for treating osteoporosis.

7. Use of Compounds of formula (I), wherein:
X is =CH₂;
one of R₁ and R₂ is hydrogen and the other is (C₁-C₄)alkyl;
R₃ and R₄ are (C₁-C₄)alkyl;
A is a double bond; and B is a double bond, particularly
wherein:
R₁ is hydrogen;
R₂ is CH₃;
R₃ and R₄ are ethyl; and
B is a trans double bond, or
wherein:
R₁ is CH₃;
R₂ is hydrogen;
R₃ and R₄ are ethyl; and
B is a trans double bond for the manufacture of a pharmaceutical composition containing such a compound of formula (I) for the treatment of osteoporosis.

8. Use of Compounds of formula (I), wherein X, R₁ to R₄, A and B are defined as in claim 1 for the manufacture of a pharmaceutical composition containing such a compound of formula (I) for the treatment of osteoporosis.

## Patentansprüche

1. Verbindung der Formel 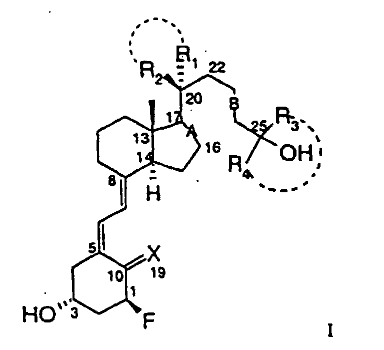 worin:
X (H,H) oder =CH₂ darstellt;
R₁ und R₂, zusammen mit C-20, eine Cyclopropan-, Cyclodifluorpropan- oder Cyclotetrafluorpropangruppe bilden;
R₃ und R₄, unabhängig voneinander, ausgewählt sind aus der Gruppe, bestehend aus CH₃, CF₃, CH₂CH₃, CH₂CF₃, CF₂CH₃ und CF₂CF₃;
A eine Einfachbindung oder eine Doppelbindung darstellt; und
B eine Doppelbindung oder eine Dreifachbindung darstellt.

2. Verbindung nach Anspruch 1, worin:
X =CH₂ darstellt;
R₁ und R₂, zusammen mit C-20, eine Cyclopropan-, Cyclodifluorpropan- oder Cyclotetrafluorpropangruppe bilden;
R₃ und R₄, unabhängig voneinander, ausgewählt sind aus der Gruppe, bestehend aus CH₃, CF₃, CH₂CH₃, CH₂CF₃, CF₂CH₃ und CF₂CF₃;
A eine Einfachbindung oder eine Doppelbindung darstellt; und
B eine Doppelbindung oder eine Dreifachbindung darstellt.

3. Verbindung nach Anspruch 2, worin:
R₁ und R₂, zusammen mit C-20, eine Cyclopropangruppe bilden; R₃ und R₄ jeweils CF₃ darstellen;
A eine Doppelbindung darstellt; und
B eine Dreifachbindung darstellt.

4. Pharmazeutische Zusammensetzung, umfassend einen pharmazeutisch verträglichen Träger und eine Verbindung der Formel (I) nach Anspruch 1.

5. Verfahren zur Herstellung von Verbindungen der Formel I nach Anspruch 1, das das Entfernen der enthaltenen Silylschutzgruppe(n) aus einer silylierten Verbindung der Formel I umfasst.

6. Verbindungen der Formel I nach Anspruch 1 zur Verwendung als therapeutisch wirksame Mittel, insbesondere zum Behandeln von Osteoporose.

7. Verwendung von Verbindungen der Formel (I), worin:
X =CH₂ darstellt;
einer von R₁ und R₂ Wasserstoff darstellt und der andere (C₁-C₄)-Alkyl darstellt;
R₃ und R₄ (C₁-C₄)-Alkyl darstellen;
A eine Doppelbindung darstellt; und
B eine Doppelbindung darstellt, insbesondere
worin:
R₁ Wasserstoff darstellt;
R₂ CH₃ darstellt;
R₃ und R₄ Ethyl darstellen; und
B eine trans-Doppelbindung darstellt, oder
worin:
R₁ CH₃ darstellt;
R₂ Wasserstoff darstellt;
R₃ und R₄ Ethyl darstellen; und
B eine trans-Doppelbindung darstellt, zur Herstellung einer pharmazeutischen Zusammensetzung, die eine Verbindung der Formel (I) enthält, zur Behandlung von Osteoporose.

8. Verwendung von Verbindungen der Formel (I), worin X, R₁ bis R₄, A und B wie in Anspruch 1 definiert sind, zur Herstellung einer pharmazeutischen Zusammensetzung, die eine solche Verbindung der Formel (I) enthält, zur Behandlung von Osteoporose.

## Revendications

1. Composé de formule 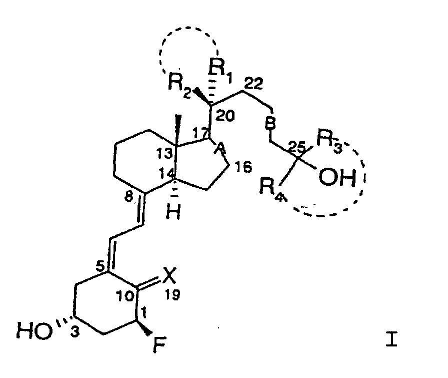 dans laquelle :
X est (H,H) ou =CH₂ ;
R₁ et R₂ forment avec C-20 un groupe cyclopropano, cyclodifluoropropano ou cyclotétrafluoropropano ;
R₃ et R₄ sont, indépendamment l'un de l'autre, choisis dans le groupe constitué par CH₃, CF₃, CH₂CH₃, CH₂CF₃, CF₂CH₃ et CF₂CF₃ ;
A est une liaison simple ou une liaison double ; et
B est une liaison double ou une liaison triple.

2. Composé selon la revendication 1, dans lequel :
X est =CH₂ ;
R₁ et R₂ forment avec C-20 un groupe cyclopropano, cyclodifluoropropano ou cyclotétrafluoropropano ;
R₃ et R₄ sont, indépendamment l'un de l'autre, choisis dans le groupe constitué par CH₃, CF₃, CH₂CH₃, CH₂CF₃, CF₂CH₃ et CF₂CF₃ ;
A est une liaison simple ou une liaison double ; et
B est une liaison double ou une liaison triple.

3. Composé selon la revendication 2, dans lequel :
R₁ et R₂ forment avec C-20 un groupe cyclopropano ; R₃ et R₄ sont tous deux CF₃ ;
A est une liaison double ; et
B est une liaison triple.

4. Composition pharmaceutique comprenant un véhicule pharmaceutiquement acceptable et un composé de formule (I) selon la revendication 1.

5. Procédé de fabrication d'un composé de formule I selon la revendication 1, lequel comprend l'étape consistant à extraire le(s) groupe(s) protecteur(s) silyle présent(s) d'un composé sylilé de formule I.

6. Composés de formule I selon la revendication 1 destinés à être utilisés en tant qu'agents thérapeutiquement actifs, en particulier pour traiter l'ostéoporose.

7. Utilisation de composés de formule (I), dans lesquels :
X est =CH₂ ;
l'un de R₁ et R₂ est l'hydrogène, l'autre étant un alkyle en (C₁-C₄) ;
R₃ et R₄ sont un alkyle en (C₁-C₄) ;
A est une liaison double ; et
B est une liaison double, en particulier
dans lesquels :
R₁ est l'hydrogène ;
R₂ est CH₃ ;
R₃ et R₄ sont l'éthyle ; et
B est une liaison double en trans, ou
dans lesquels :
R₁ est CH₃ ;
R₂ est l'hydrogène ;
R₃ et R₄ sont l'éthyle ; et
B est une liaison double en trans pour la fabrication d'une composition pharmaceutique contenant un tel composé de formule (I) pour le traitement de l'ostéoporose.

8. Utilisation de composés de formule (I), dans lesquels X, R₁ à R₄, A et B sont tels que définis dans la revendication 1 pour la fabrication d'une composition pharmaceutique contenant un tel composé de formule (I) pour le traitement de l'ostéoporose.
